# EUROPEAN PATENT APPLICATION

(11) **EP 4 393 548 A2**
(43) Date of publication of application: **03.07.2024**
(21) Application number: 24168102.2
(22) Date of filing: 02.05.2017
(51) Int. Cl.: A61P 39/04

(54) **FORMULATIONS AND TREATMENTS EMPLOYING HYDROXYPYRIDONATE ACTINIDE/LANTHANIDE DECORPORATION AGENTS**

(30) Priority: 03.05.2016 US 201662331369 P; 06.09.2016 US 201662383963 P
(62) Divisional of application: 17793154.0
(71) Applicant: The Regents of the University of California, Oakland, CA 94607 (US)
(72) Inventor: ABERGEL, Rebecca J., Oakland, 94607 (US); DEBLONDE, Gauthier J.P., Oakland, 94607 (US)
(74) Representative: Arth, Hans-Lothar

(57) **Abstract**

Provided herein are methods for treating a subject in need of such treatment comprising administering a therapeutically effective amount of one or more pharmaceutical compositions comprising a 1,2-HOPO chelating agent and/or a 3,2-HOPO chelating agent to a subject in need of such treatment. The subject can be one who has been, or will be, exposed to one or more known or unknown actinides and/or lanthanides, or a mixture thereof.

## Description

### STATEMENT OF GOVERNMENTAL SUPPORT

The invention was made with government support from the National Institutes of Health (NIAID/NIH, Grant #RAI087604Z) through the U.S. Department of Energy Laboratory under Contract No. DE-AC02-05CH11231. The government has certain rights in the invention.

### BACKGROUND OF THE INVENTION

### Field of the Invention

This invention relates generally to the treatment of metal poisoning.

### Description of the Related Art

Exposure to radionuclides accidentally or deliberately scattered by a radiological dispersion device or deposited from a nuclear power plant accident or nuclear device detonation could result in the contamination of a large population. As internalized radionuclides are highly toxic and may cause both acute and chronic radiation injury, such contamination event would have dramatic public health consequences.

Decorporation by chelating agents is a way to reduce exposure of certain incorporated isotope, and diethylenetriaminepentaacetic acid (DTPA) has been a standard therapy for actinide/lanthanide decorporation since its development and use by the U.S. Atomic Energy Commission in the 1950's.

### SUMMARY OF THE INVENTION

Embodiments herein provide for a method for treating a subject in need of such treatment comprising administering a therapeutically effective amount of one or more pharmaceutical compositions comprising a 1,2-HOPO chelating agent and a 3,2-HOPO chelating agent to a subject in need of such treatment. This is especially useful when practiced on a subject that has been, or will be, exposed to, in contact with, or contaminated by one or more known or unknown actinides and/or lanthanides, or a mixture thereof.

In some embodiments, a method for treating a subject for a heavy metal exposure is provided. The method comprises administering a therapeutically effective amount of a pharmaceutical composition comprising a 1,2-HOPO chelating agent to a subject that has an excess amount of one or more of gadolinium, lead, tin, yttrium, scandium, or cadmium, wherein administering results in decorporating, clearing or reducing the amount of gadolinium, lead, tin, or cadmium from the subject. In some embodiments, the subject has been exposed to, have been in contact with, or contaminated by one or more actinides and/or lanthanides, or a mixture thereof.

In some embodiments, the administering step results in decorporating, clearing or reducing the amount of actinide and/or lanthanide, or both from one or more systems or organs of the subject.

In some embodiments, the 1,2-HOPO chelating agent is defined by the structure: 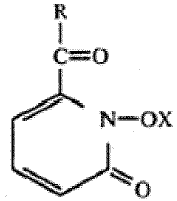 wherein R is a hydroxy group or 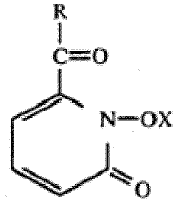 where R₁ and R₂ are selected from the group consisting of H, --CH₃,-CH₂CH₃ and --CH₂--ϕ, and X is either hydrogen, an alkali metal ion, or a quaternary ammonium ion.

In some embodiments, the 1,2-HOPO chelating agent is defined by one molecule selected from the group consisting of: 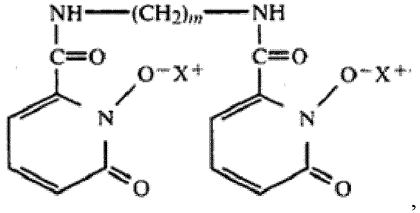 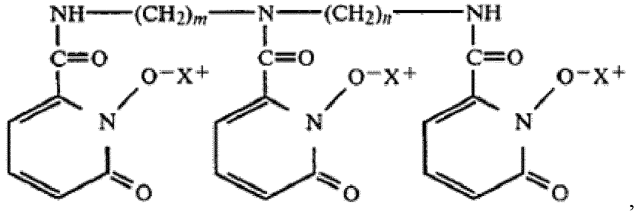 and 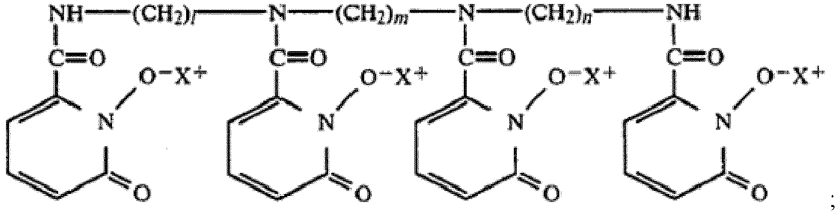 wherein l, m and n are integers between one and twenty. In some embodiments, m is three. In some embodiments, n is four. In some embodiments, l and n are three, and m is four. In some embodiments, the 1,2-HOPO chelating agent is 3,4,3-LI-1,2-HOPO. In some embodiments, the subject has an excess amount of one or more of gadolinium, lead, yttrium, scandium, cadmium, or tin.

In some embodiments, a method for the prophylactic treatment of a subject for metal exposure is provided. The method comprises administering a therapeutically effective amount of a pharmaceutical composition comprising a 1,2-HOPO chelating agent to a subject. In some embodiments, the 1,2-HOPO chelating agent is 3,4,3-LI-1,2-HOPO. In some embodiments, the metal is a heavy metal. In some embodiments, the heavy metal is selected from the group consisting of gadolinium, lead, tin, cadmium, yttrium, scandium, and plutonium. In some embodiments, the metal is an actinide a lanthanide, or a mixture thereof.

In some embodiments, a method is provided that comprises administering a 1,2-HOPO chelating agent to a subject prior to or after administering a MRI contrast agent to the subject. In some embodiments, the subject is identified as one that is to receive the MRI contrast agent. In some embodiments, the contrast agent is comprises Gd. In some embodiments, an amount of the contrast agent is from 100-600 µmol/kg. In some embodiments, the 1,2-HOPO chelating agent is administered prior to the subject receiving the MRI contrast agent. In some embodiments, the 1,2-HOPO chelating agent is administered after the subject received the MRI contrast agent. In some embodiments, the 1,2-HOPO chelating agent is 3,4,3-LI-1,2-HOPO. In some embodiments, the subject has severely impaired kidney function.

### BRIEF DESCRIPTION OF THE DRAWINGS

The foregoing aspects and others will be readily appreciated by the skilled artisan from the following description of illustrative embodiments when read in conjunction with the accompanying drawings.

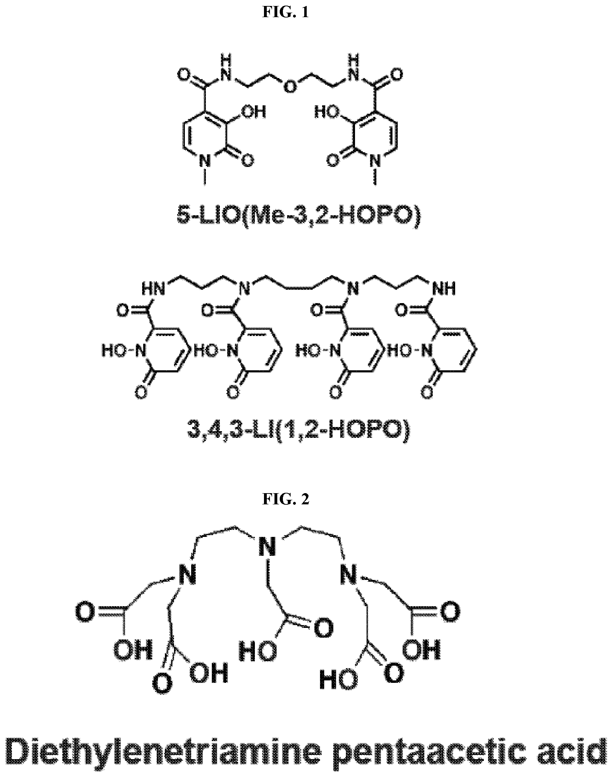

FIG. 1 shows the structures of 5-LIO(Me-3,2-HOPO) ("SLIO") and 3,4,3-LI(1,2-HOPO) ("343LI").
FIG. 2 shows the structure of diethylenetriamine pentaacetic acid (DTPA).
FIG. 3 depicts the total ²³⁸Pu body content and distribution at 7 days after a single time-delayed ip chelation treatment.

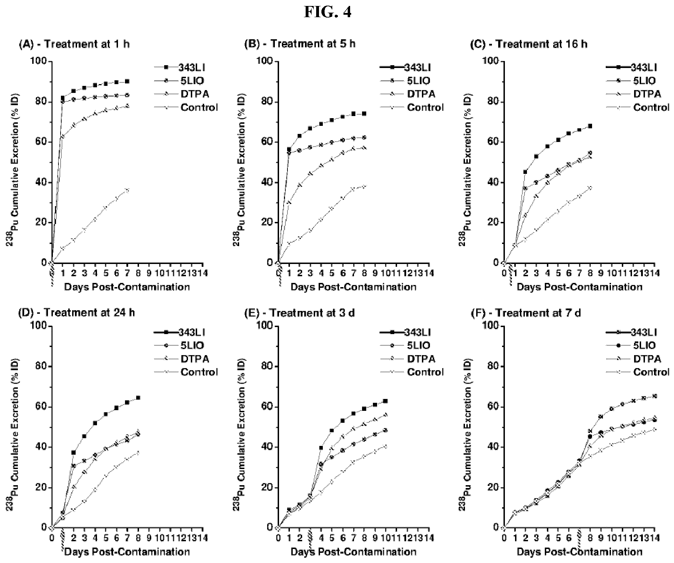

FIG. 4 depicts the daily ²³⁸Pu cumulative excretion after a single time-delayed ip chelation treatment at 1 h (**A**), 5 h (**B**), 16 h (**C**), 24 h (**D**), 3 d (**E**), or 7 d (**F**) post-contamination. Young adult female Swiss-Webster mice injected iv with ²³⁸Pu-citrate; saline or treatment (3,4,3-LI(1,2-HOPO) [30 µmol/kg], 5-LIO(Me-3,2-HOPO) [100 µmol/kg], or Ca-DTPA [30 µmol/kg]) administered ip at 1 h, 5 h, 16 h, 24 h, 3 d, or 7 d post-contamination as indicated by the arrows; mice euthanized 7 days after treatment. Excreta of each five-mouse group were pooled and standard deviations are not available as the data are calculated from the cumulative collected excretion.
FIG. 5 depicts the daily ²³⁸Pu fecal (left panels **A, C, E, G)** and urinary (right panels **B, D, F, H)** output after a single 3,4,3-LI(1,2-HOPO) **(A** and **B),** 5-LIO(Me-3,2-HOPO) **(C** and **D),** DTPA **(E** and **F),** or saline **(G** and **H),** time-delayed ip chelation treatment at 1 h, 5 h, 16 h, 24 h, 3 d, or 7 d post-contamination.
FIG. 6A-C depict total ²³⁸Pu body content and distribution 3 days after a contamination event preceded by a single prophylactic chelation treatment.
FIG. 7 depicts daily ²³⁸Pu fecal (top panels **A** and **B)** and urinary (bottom panels **C** and **D)** output after a single 3,4,3-LI(1,2-HOPO) prophylactic ip (left panels **A** and **C)** or po (right panels B and **D)** chelation treatment.
FIGs. 8A-8Q depict the results from Example 9. The results are plotted as a percent of recovered dose (percent RD).

### DETAILED DESCRIPTION OF VARIOUS EMBODIMENTS

The potential consequences of a major radiological event are not only large-scale external radiation exposure of the population, but also uncontrolled dissemination of, and internal contamination with, radionuclides. When planning an emergency response to radiological and nuclear incidents, one must consider the need for not only post-exposure treatment for contaminated individuals, but also prophylactic measures to protect the workforce facing contaminated areas and patients in the aftermath of such events. In addition to meeting the desired criteria for post-exposure treatments such as safety, ease of administration, and broad-spectrum efficacy against multiple radionuclides and levels of challenge, ideal prophylactic countermeasures must include rapid onset; induce minimal to no performance-decrementing side effects; be compatible with current military Chemical, Biological, Radiological, Nuclear, and Explosive countermeasures; and require minimal logistical burdens.

Hydroxypyridinone-based actinide decorporation agents have shown the promise as decorporation strategies for various radionuclides of concern, including the actinides plutonium and americium. Some of the results presented here probe the extent of plutonium decorporation efficacy for two chelating agents, 3,4,3-LI(1,2-HOPO) and 5-LIO(Me-3,2-HOPO), from early pre-exposure time points to a delay of up to 7 days in parenteral or oral treatment administration, i.e., well beyond the first hours of emergency response. Despite delayed treatment after a contamination event, both ligands clearly enhanced plutonium elimination through the investigated 7-day post-treatment period. In addition, a remarkable prophylactic efficacy was revealed for 3,4,3-LI(1,2-HOPO) with treatment as early as 48 hours before the plutonium challenge. This work provides new perspectives in the indication and use of experimental actinide decorporation treatments.

Prompt decorporation is crucial for mitigating both immediate and future biological effects from radiological contamination. Adverse health effects include tissue damage and the development of various cancers, and are dependent upon factors such as the quantity of contaminants and duration of contamination. Internal contamination, i.e., the deposition of radionuclides in the body via routes that include ingestion, inhalation, and absorption through wounds, is especially dangerous since it may produce local, systemic, or a combination of radiation effects.

To enhance emergency preparedness in the United States in response to potential nuclear accidents and terrorist threats, the U.S. Food and Drug Administration (FDA) approved two forms of diethylenetriamine pentaacetic acid (DTPA) in 2004calcium (Ca-DTPA) and zinc (Zn-DTPA) -- to expedite the excretion of plutonium, americium, and curium after internal contamination. Although it is the first and only drug approved for treating internal contamination with the aforementioned radioactive elements, DTPA's efficacy is limited to certain forms of these elements, routes of administration, and dosages. The drug's efficacy is hindered when isotopes are mixed with other materials; as a result of its low absorption in the gastrointestinal tract, it needs to be administered either intravenously or via nebulized inhalation depending on the route of contamination; and it must be taken in large quantities. Experiments have also shown that Ca-DTPA does not chelate plutonium significantly after the element's deposition in organs, explaining the necessity of administering treatment as soon as possible post-contamination. However, although the large molar percentage of DTPA administered parenterally can be accounted for in blood and extracellular fluid, a small fraction can reach intracellular spaces responsible for the liver decorporation efficacy, as demonstrated in rats and dogs. Additionally, DTPA's side effects include the loss of essential metals such as zinc and magnesium from the body, further emphasizing the need for alternative decorporation therapy.

Addressing the limitations of Ca-DTPA and Zn-DTPA, an octadentate hydroxypyridinone-based chelator, 3,4,3-LI(1,2-HOPO), has shown efficacy with high potency and low toxicity through parenteral and oral routes of administration, preferred qualities in drug development. Studies have not only considered sex bias by examining efficacy in both male and female mice, but also elucidated the ability of 3,4,3-LI(1,2-HOPO) to, at physiological pH, form stable, excretable complexes with those radiological elements chelated by DTPA along with others such as isotopes of uranium, neptunium, and europium. Its efficacy and safety have been proven in multiple animal models in order to meet criteria in the FDA's *Animal Efficacy Rule* and gain approval since efficacy trials in human beings cannot be ethically conducted. A promising candidate for treating internal radionuclide contamination, 3,4,3-LI(1,2-HOPO) received an investigational new drug (IND) designation from the FDA in August 2014 and is awaiting phase I clinical trials.

The experiments described herein identify the potential of delayed and prophylactic treatments via intraperitoneal injection or oral administration for internal plutonium contamination. Realistically, treatment for the majority of the population following a radiological incident will not be accessible until after the first 24 hours of emergency response; likewise, prophylaxis is crucial for first-responders and the military. Ideal prophylaxis should provide broad-spectrum protection against multiple isotopes and levels of challenge. In some embodiments, it is also desirable for it to be safe, efficacious, have a rapid onset, be easily administered, induce minimal to no performance-decrementing side effects, be compatible with current military Chemical, Biological, Radiological, and Nuclear (CBRN) countermeasures, and/or require minimal logistical burdens. Consequently, the current limitations of DTPA-based products and the lack of a viable drug for use prior to exposure to radiological material stress the significance and urgency of developing novel, efficacious decorporation therapies such as 3,4,3-LI(1,2-HOPO). In addition, other embodiments and applications for chelators are also provided herein, such as their use in reducing risks associated with MRI contrast agents.

The following disclosure provides a brief set of definitions, then provides further detail regarding the various embodiments for treatment, prophylactic and otherwise, involving the chelators provided herein, and then provides a set of Examples regarding various embodiments.

### Definitions

The term "emergency" encompasses: (a) The event of an accidental release of the radioisotopes in the environment due to any nuclear accident. (b) Any accidental release of the hazardous nuclides in the environment. (c) A nuclear fallout including that occurring in the normal course of an experimental, diagnostic or therapeutic purpose. (d) Any kind of accidental uptake and retention of the radionuclides by the human or animal subjects. (e) Any other kind of exposure to the volatile radionuclides. (f) Any kind of a radiological accident.

The term "pharmaceutically acceptable salt," as used herein, and particularly when referring to a pharmaceutically acceptable salt of a compound, including 3,4,3-LI(1,2-HOPO), and refers to any pharmaceutically acceptable salts of a compound, and preferably refers to an acid addition salt of a compound.

The terms "pure," "purified," "substantially purified," and "isolated" as used herein refer to the compound of the embodiment being free of other, dissimilar compounds with which the compound, if found in its natural state, would be associated in its natural state. In certain embodiments described as "pure," "purified," "substantially purified," or "isolated" herein, the compound can comprise at least 0.5% to 1%, 1% to 5%, 5% to 10%, 10% to 20%, 20% to 50%, 50% to 70%, 70% to 90%, 90% to 95%, 95% to 99%, and 99% to 100%. In some embodiments, the amount of the compound will be at least 50% or 75% of the mass, by weight, of a given sample. A "functional purity" is a measurement of the amount of a particular compound in a sample or product in relation to other compounds in a sample that can adversely impact the function of the compound. Thus, other components in a sample that do not interfere with the compound's activity (e.g., water), will not be used in determining the purity of a sample or product.

The terms "derivative," "variant," or other similar term refers to a compound that is an analog of the other compound.

The term "and/or" designates both the option of "and" as well as the option of "or" in that particular circumstance. However, unless otherwise specified in the specification, the use of the term "or" or "and" encompasses a description of both option as well. Thus, the use of the term "or" should not be taken as excluding the option of "and", unless additional context indicates that it should (this definition does not apply to the language in the claims). The use of the singular or plural forms of a term encompasses both options (singlular or plural) as well as both options combined (singular and plural), unless indicated otherwise.

The term "inhibition" as used herein, refers to any statistically significant decrease in the detrimental impact of the metal, including full blocking of the activity. For example, "inhibition" can refer to a decrease of about 10%, 20%, 30%, 40%, 50%, 60%, 70%, 80%, 90%, or 100% in the detrimental impact of the metal.

The term "patient" includes human and other mammalian subjects that receive either prophylactic or therapeutic treatment.

The terms "treat" or "prevent" do not require complete treatment or complete prevention under all conditions. A slowing of the onset of a disorder or its symptoms or a decrease in the number of the symptoms can be adequate "prevention" in some embodiments. Similarly, a decrease in the severity of the symptoms of the disorder can also be an effective treatment for a disorder. "Prophylactic treatment" denotes that the compound is administered prior to exposure to the detrimental compound (e.g., metal such as plutonium or a MRI imaging agent). Treatment may also be in response to exposure, e.g., responsive therapy. Treat also encompasses remediation, decorporation, and/or decontamination.

"Therapeutically effective amount" means that amount of the chelating agents, such as 3,4,3-LI(1,2-HOPO), 5-LIO(Me-3,2-HOPO) and /or DTPA, that elicit the biological or medicinal response in a tissue system, animal or human sought by a researcher, veterinarian, medical doctor or other clinician, which response includes alleviation of the symptoms of the disease or disorder being treated. The specific amount of chelating agents needed to elicit the biological or medicinal response will depend on a number of factors, including but not limited to the disease or disorder being treated, the chelating agents being administered, the method of administration, and the condition of the patient.

"Mammal" when used herein refers to any animal that is considered a mammal. Preferably, the mammal is human.

The term "pharmaceutical agent or drug" as used herein refers to a chemical compound or composition capable of inducing a desired therapeutic effect when properly administered to a patient. Other chemistry terms herein are used according to conventional usage in the art, as exemplified by *The McGraw-Hill Dictionary of Chemical Terms* (Parker, S., Ed., McGraw-Hill, San Francisco (1985)), (incorporated herein by reference).

The term "heavy metal" denotes one or more of a transition metal, a metalloid, a metallic element within groups 13, 14, and 15 of the Periodic Table, an actinide and/or a lanthanide. Heavy metals include, for example, gadolinium, lead, tin, cadmium, yttrium, scandium, and plutonium.

### Methods Employing Chelators

Previous research has characterized radionuclide elimination enhancement after prompt or slightly delayed treatment with chelating agents, most often within the first 24 hours of the contamination event. Scenarios of human contamination with actinides include a myriad of unknowns, but it unfortunately seems improbable that treatment would undergo wide distribution and be made available to a large population immediately post-incident.

One of the experiments provided in the Examples below establishes the extent of decorporation efficacy that is well beyond the first 24 hours of emergency response, with a delay of up to 7 days in treatment administration. In this case, extending the necropsy time points to 7 days post treatment was useful for the comparison of plutonium excretion patterns over several days, i.e. until rates of actinide elimination slowed considerably. When preparing for a nuclear emergency, further considerations must be taken into account, including the need for pre-event prophylaxis, particularly for the military and first-responders. In another study presented below, a different experimental protocol was adopted to explore the potential prophylactic activity of the actinide decorporation agents. Treatment was administered either parenterally or orally at times preceding the ²³⁸Pu challenge, with a delay of up to 48 hours prior to contamination. In this study, the necropsy time point was set at 3 days post-contamination, independent of the treatment time.

When provided after the contamination, and despite delayed treatment, parenteral 3,4,3-LI(1,2-HOPO), 5-LIO(Me-3,2-HOPO) and DTPA all clearly enhanced ²³⁸Pu elimination through the investigated 7-day post-treatment period.

However, the two HOPO ligands behaved distinctly in that the sustained excretion enhancement known and confirmed for 3,4,3-LI(1,2-HOPO) and DTPA over several days was not observed with 5-LIO(Me-3,2-HOPO). Furthermore, the rates of ²³⁸Pu elimination observed after 5-LIO(Me-3,2-HOPO) or DTPA treatment slowed down earlier than those following 3,4,3-LI(1,2-HOPO) injections.

The superiority of 3,4,3-LI(1,2-HOPO) in enhancing ²³⁸Pu rates of elimination was also evidenced in the prophylactic protocol: while 5-LIO(Me-3,2-HOPO) and DTPA resulted in slight ²³⁸Pu content reduction if administered within 6 hours prior to contamination, the efficacy of 3,4,3-LI(1,2-HOPO) was remarkably higher and observed even with treatments as early as 48 hours before the ²³⁸Pu challenge.

While the ²³⁸Pu complexes formed with 3,4,3-LI(1,2-HOPO) or DTPA undergo relatively fast clearance and are fully expelled from the body within 24 hours, it has been suggested that the sustained excretion enhancement profiles in treated animals are due to intracellular uptake of the ligands and delayed clearance. The pharmacokinetics and biodistribution profile of the ¹⁴C-labeled 3,4,3-LI(1,2-HOPO) were recently characterized in the young Swiss-Webster mouse model. After parenteral injection, the radiolabeled compound was rapidly distributed into high vascular tissues, and highest kidney and liver concentrations were seen as early as 1-hour post-administration. However, those high concentrations stayed constant and more strikingly, about 40% of the administered dose was still in various tissues and organs after 24 hours. These results were a good indicator of a longer residence time for 3,4,3-LI(1,2-HOPO) and correlate well with both its sustained action after treatment and large prophylactic window. Similarly, clearance was faster after oral administration of the radiolabeled ligand, as most of the compound remained unabsorbed and was simply excreted by 24 hours. Nevertheless, retention of the ligand was significant within the first 6 hours after oral administration, which also corroborates the shorter but consequent prophylactic activity of 3,4,3-LI(1,2-HOPO) after oral administration.

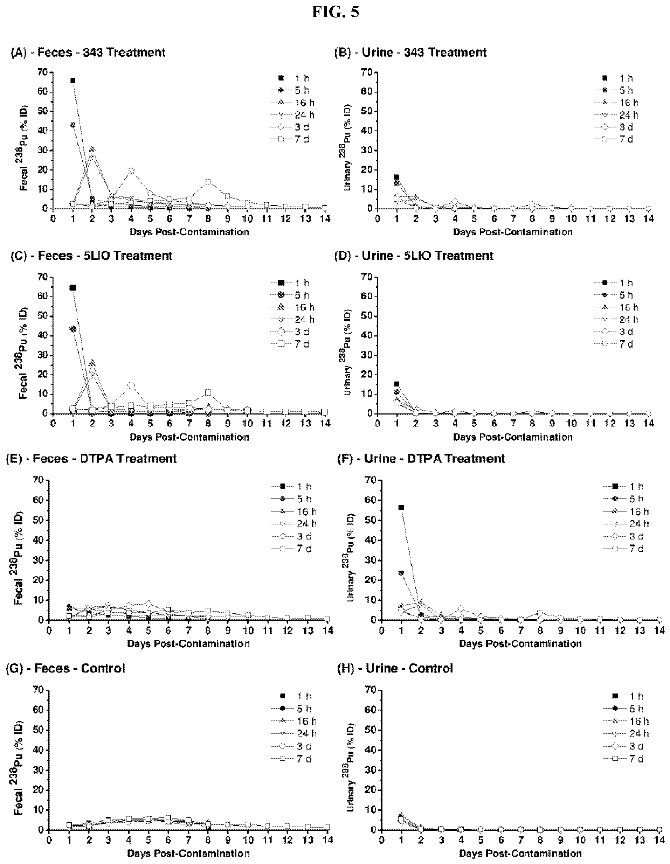

The predominant biliary pathway observed in ²³⁸Pu excretion promoted by either HOPO ligand is drastically different from the enhanced urinary excretion patterns resulting from treatment with DTPA, as evidenced by the illustration of the fecal and urinary ²³⁸Pu outputs in FIG. 5. Those differences have been discussed previously and are presumably based on respective ligand and actinide-complex physico-chemical parameters such as solubility, lipophilicity, and ionization constants. In addition, the biliary pathway is the main mode of elimination for 3,4,3-LI(1,2-HOPO) in mice, as demonstrated by high accumulation of ¹⁴C-labeled 3,4,3-LI(1,2-HOPO) in the feces after either parenteral or oral administration. It is therefore likely that the radionuclide excretion path is ligand-driven. Of particular interest is the fact that a ²³⁸Pu urinary output 3-fold higher than the fecal level was noted in the single case of 3,4,3-LI(1,2-HOPO) prophylactic parenteral treatment 1 hour prior to contamination. The disposition profiles of the ¹⁴C-labeled ligand also displayed higher urinary excretion at early time points, within 4 hours of ligand administration, due to the longer colonic transit times associated with biliary excretion. The excretion pattern observed in the early prophylactic case therefore suggests that the 3,4,3-LI(1,2-HOPO) ligand chelated the available ²³⁸Pu fraction that had just entered the systemic circulation, preventing it from reaching the radionuclide deposition sites.

As detailed above, and demonstrated in the Examples below, ²³⁸Pu elimination enhancement was observed after parenteral 3,4,3-LI(1,2-HOPO), 5-LIO(Me-3,2-HOPO), or DTPA treatments delayed to up to 7 days post-contamination, with roughly equivalent efficacies for the two latter ligands but large and significant reductions in full body and tissue content in the case of 3,4,3-LI(1,2-HOPO). However, decorporation efficacy clearly decreases with increasing delays in treatment. Another attribute of the 3,4,3-LI(1,2-HOPO) ligand uncovered in the studies provided herein is its high decorporation efficacy when administered prophylactically as early as 48 hours pre-exposure. While, it is remarkable that the lead hydroxypyridinonate chelating agent under development, 3,4,3-LI(1,2-HOPO) promotes significant removal of ²³⁸Pu from contaminated mice even when a single treatment is delayed to 7 days after exposure, the results of this study confirm that decorporation treatments should be administered with the shortest possible delay post-contamination. In addition, the long prophylactic window of activity revealed here opens a new perspective in the indication and use of this decorporation treatment. Extensive non-clinical studies have highlighted the safety of hydroxypyridinonate chelating agents and, in the absence of serious toxicity concerns, it is now a possibility that 3,4,3-LI(1,2-HOPO) be considered useful for prophylactic use, in which treatment decisions will be made based on suspicion or prevention of potential contamination rather than on knowledge that may require long triage, estimation, and decision-making procedures in emergency settings. These results therefore complements the available efficacy data sets for hydroxypyridinonate ligands in general and 3,4,3-LI(1,2-HOPO) in particular, and combined with parallel clinical safety analyses, will help define therapeutic options for ²³⁸Pu decorporation.

Various applications of the above aspects are provided below.

### Methods of Use

In some embodiments, a method for treating a subject for a heavy metal exposure is provided. The method comprises administering a therapeutically effective amount of a pharmaceutical composition comprising a 1,2-HOPO chelating agent to a subject that has an excess amount of one or more of gadolinium, lead, tin, yttrium, scandium, and/or cadmium. The administration results in decorporating, clearing or reducing the amount of gadolinium, lead, tin, and/or cadmium from the subject.

In some embodiments, the subject has been exposed to and/or has been in contact with, and/or will be exposed to or contaminated by one or more actinides and/or lanthanides, or a mixture thereof. In some embodiments, the subject has an excess amount of one or more of gadolinium, lead, yttrium, scandium, cadmium, or tin. In some embodiments, the subject has an excess amount of gadolinium. In some embodiments, the subject has an excess amount of lead. In some embodiments, the subject has an excess amount of yttrium. In some embodiments, the subject has an excess amount of scandium. In some embodiments, the subject has an excess amount of cadmium. In some embodiments, the subject has an excess amount of tin. An excess amount can be an amount that is beyond a normal baseline or background level. In some embodiments, an excess amount can be an amount that is unhealthy for the subject. In some embodiments, the excessive amount is at least 1, 10, 20, 30, 40, 50, 60, 70, 80, 90, 100, 1000, 10,000 percent or more than the amount of the heavy metal that is present in an unexposed individual. That is, it is at least 1, 10, 20, 30, 40, 50, 60, 70, 80, 90, 100, 1000, 10,000 percent higher than baseline levels that are due to a standard environment. In some embodiments, such as the case of Gd, average doses for different contrast agents are 0.1 mmol/kg, or 7 mmol for a 70 kg adult, which is roughly 1 g of Gd per patient. For some MRI scans, patients receive about 1 g of chelated Gd, and some % of that Gd remains in the body. Thus, reducing the amount of Gd that remains in the body beneath this level can be advantageous to the subject. For lead (Pb), the excess amount can be a much lower level, as it is more toxic.

In some embodiments, the method is prophylactic. The method for the prophylactic treatment of a subject for metal exposure can comprise administering a therapeutically effective amount of a pharmaceutical composition comprising a 1,2-HOPO chelating agent to a subject.

In some embodiments, the chelating agent can be any provided herein. In some embodiments, the 1,2-HOPO chelating agent is 3,4,3-LI-1,2-HOPO. In some embodiments, a 3,4,3-LI-1,2-HOPO compound can be used for the treatment of heavy metal exposure, either before or after exposure. In some embodiments, one can use 3,4,3-LI-1,2-HOPO in the preparation of a medicament for the treatment of heavy metal exposure, either pre- or post-exposure to the heavy metal. In some embodiments, the composition can comprise 3,4,3-LI-1,2-HOPO for the treatment of heavy metal exposure, either before or after exposure. In some embodiments, the composition can comprise 3,4,3-LI-1,2-HOPO for neutralizing contrast agents from MRIs, such as a Gadolinium-based contrast agent. In some embodiments, one can use 3,4,3-LI-1,2-HOPO for the preparation of a medicament for reducing and/or removing a Gadolinium-based contrast agent (GBCA).

In some embodiments, the subject has been, or will be, exposed to one or more of a heavy metal. In some embodiments, the subject is scheduled or is about to receive an MRI. In some embodiments, the MRI procedures necessitate administration of a Gadolinium-based contrast agent (GBCA), such as gadoterate (Dotarem), gadodiamide (Omniscan), gadobenate (MultiHance), gadopentetate (Magnevist), gadoteridol (ProHance), gadofosveset (Ablavar, formerly Vasovist), gadoversetamide (OptiMARK), gadoxetate (Eovist), or gadobutrol (Gadavist). In some embodiments, one or more negative effects from the MRI contrast agent can be reduced by the use, prophylactic or otherwise, of one or more of the chelators provided herein, and 3,4,3-LI-1,2-HOPO in particular. In some embodiments, the contrast agent comprises Gd. In some embodiments, effects include: 1) Pain - aching; burning, tingling, and/or prickling pain (paresthesia); deep bone pain; typically in extremities or joints, and sometimes in the location where the MRI occurred, like the head; 2) Dermal changes - like tight skin, lesions, hyperpigmentation; most often in extremities; 3) Muscle issues - twitching - small, local, rapid contractions and weakness; 4) Ocular problems - worsening vision, dry eyes, bloodshot eyes; 5) Cognitive symptoms; 6) Ear, nose and throat - tinnitus, swallowing, and voice problems; 7) Low body temperature; 8) Hair loss; 9) Itchy skin; 10) Balance problems; 11) Swelling of extremities (edema); and/or 12) a sense of an electrified, vibrating, twitching feeling typically just under the skin that is sometimes localized and at other times a more overall feeling.

In some embodiments, the administering step results in decorporating, clearing or reducing the amount of actinide and/or lanthanide, or both from one or more systems and/or organs of the subject. In some embodiments, there is a reduction in the heavy metal present in the subject by at least 1, 2, 3, 4, 5, 10, 15, 20, 25, 30, 35, 40, 45, 50, 55, 60, 65, 70, 75, 80, 85, 90, 95, 96, 97, 98, 99, or 100 percent. In some embodiments, when applied prophylactically, the heavy metal passes through the subject more quickly, and need not actually accumulate in the subject in order for the method to be beneficial. In some embodiments, the heavy metal is present in the subject 10, 20, 30, 40, 50, 60, 70, 80, or 90% less time, than if the subject had not received the chelator. In some embodiments, the heavy metal is excreted from the subject 1.1, 2, 3, 4, 5, 6, 7, 8, 9, 10, 100, 1000, 10,000 or more fold faster than if the subject had not received the chelator.

In some embodiments, the chelator is administered 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48 or more hours prior to the subject being exposed to or being at risk of exposure to a heavy metal as described herein.

In some embodiments, the metal is a heavy metal. In some embodiments, any of the heavy metals provided herein can have its detrimental impact on health reduced by one or more the chelators provided herein in a prophylactic manner. In some embodiments, the heavy metal is selected from at least one of the group consisting of gadolinium, lead, tin, cadmium, yttrium, scandium, and plutonium. In some embodiments, the prophylactic chelating agent can be used for protection against a heavy metal, an actinide, a lanthanide, or a mixture thereof.

In some embodiments, the 1,2-HOPO chelating agent is defined by the structure: 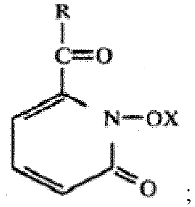 wherein R is a hydroxy group or 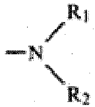 where R₁ and R₂ are selected from the group consisting of H, --CH₃, --CH₂CH₃ and --CH₂--ϕ, and X is either hydrogen, an alkali metal ion, or a quaternary ammonium ion.

In some embodiments, the 1,2-HOPO chelating agent is defined by one molecule selected from the group consisting of: 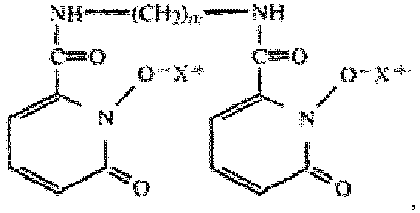 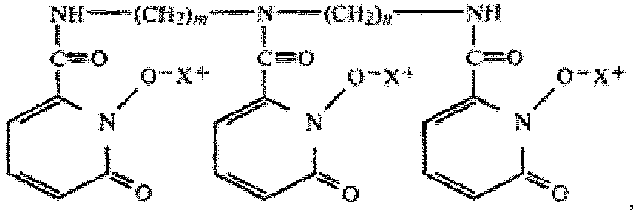 and 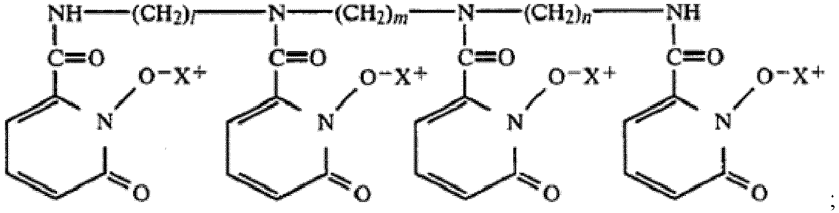 wherein l, m and n are integers between one and twenty.

In some embodiments, m is three and/or n is four. In some embodiments, l and n are three, and m is four. In some embodiments, the 1,2-HOPO chelating agent is 3,4,3-LI-1,2-HOPO.

Suitable 3,2-HOPO chelating agents include, but are not limited to, a chelating agent having the structure:

### Modes of Administration and Pharmaceutical Formulations

Suitable modes of administration of the pharmaceutical composition include, but are not limited to, oral, topical, aerosol, inhalation by spray, parenteral, subcutaneous, intravenous, intramuscular, interperitoneal, rectal, and vaginal administration. The term parenteral, as used herein, includes subcutaneous injections, and intravenous, intrathecal, intramuscular, and intrasternal injection or infusion techniques. A particular mode of administration is one that brings a chelating agent to the actual or potential site(s) of radionuclide contamination in the subject. The pharmaceutical composition can be in a solid, semi-solid, and/or liquid form. In some embodiments, any of the above formulations can be used for any of the metals provided herein (including gadolinium, lead, tin, and/or cadmium) and/or for prophylactic use.

The pharmaceutically acceptable carriers described herein, for example, vehicles, adjuvants, excipients, and diluents, are well known to those who are skilled in the art and are readily available. In some embodiments, the carrier is chemically inert to a compound of this chelating agent and has no detrimental side effects or toxicity under the conditions of use. In some embodiments, the pharmaceutically acceptable carrier is free of pyrogen. The pharmaceutically acceptable carriers which can be used include, but are not limited to, water, glucose, lactose, gum acacia, gelatin, mannitol, starch paste, magnesium trisilicate, talc, corn starch, keratin, colloidal silica, potato starch, and urea.

The amount of the chelating agents that may be combined with the pharmaceutically acceptable carrier to produce a single dosage form will vary depending upon the subject treated and the particular mode of administration. Suitable dosage levels of the chelating agents include from about 1 mg to about 500 mg per kg body weight per day. In some embodiments, the suitable dosage level is from about 20 mg to about 100 mg per kg body weight per day. In some embodiments, the suitable dosage level is from about 10 µmol to about 100 µmol per kg body weight for 3,4,3-LI-1,2-HOPO. In some embodiments, the suitable dosage level is from about 30 µmol to about 200 µmol per kg body weight for 5-LIO-Me-3,2-HOPO. Dosage unit forms will generally contain from about 20 mg to about 100 mg of the chelating agents. In addition, the pharmaceutical composition can be administered on an intermittent basis, i.e., at daily, semi-weekly, or weekly intervals. It will be understood, however, that the specific dose level for a particular subject will depend on a variety of factors. These factors include the activity of the specific compound employed; the age, body weight, general health, sex, and diet of the subject; the time and route of administration and the rate of excretion of the chelating agents; the combination of chelating agents employed in the treatment; and, the severity of the particular disease or condition for which therapy is sought.

The pharmaceutical compositions suitable for oral administration include, but are not limited to, (a) liquid formulations; (b) capsules, sachets, tablets, lozenges, and troches, each containing a predetermined amount of the active ingredient, as solids or granules; (c) powders; (d) suspensions; and (e) suitable emulsions. Liquid formulations may include diluents, such as water and alcohols, and optionally a pharmaceutically acceptable surfactant. Capsule forms can be of the ordinary hard- or soft-shelled gelatin type containing, for example, surfactants, lubricants, and inert fillers. Tablet forms can include one or more of lactose, sucrose, mannitol, corn starch, potato starch, alginic acid, microcrystalline cellulose, acacia, gelatin, guar gum, colloidal silicon dioxide, croscarmellose sodium, talc, magnesium stearate, calcium stearate, zinc stearate, stearic acid, and the like. The tablet can further comprise one or more colorants, diluents, buffering agents, disintegrating agents, moistening agents, preservatives, or flavoring agents.

The pharmaceutical composition, alone or in combination with other suitable components, can be made into aerosol formulations to be administered via inhalation. These aerosol formulations can be placed into pressurized acceptable propellants (such as dichlorodifluoromethane, propane, nitrogen, and the like) or non-pressured preparations (such as in a nebulizer or an atomizer). When the site(s) of infection of a subject is the lungs, a preferred mode of administration is inhalation of an aerosol formulation either orally or nasally. in particular, the aerosol formulation may comprises particles of a respirable size, including, but not limited to, mean particle sizes of 5 µm to 500 µm.

The pharmaceutical composition can be an injectable formulation. The requirements for effective carriers for injectable compositions are well known to those of ordinary skill in the art (see, e.g., Pharmaceutics and Pharmacy Practice, J. B. Lippincott Company, Philadelphia, Pa., Banker and Chalmers, eds., pages 238-250 (1982), and ASHP Handbook on Injectable Drugs, Toissel, 4th ed., pages 622-630 (1986)). In particular embodiments, injectable compositions are administered intravenously. Formulations suitable for parenteral administration include aqueous and non-aqueous, isotonic sterile injection solutions, which can contain anti-oxidants, buffers, bacteriostats, and solutes that render the formulation isotonic with the blood of the intended recipient, and aqueous and non-aqueous sterile suspensions that can include suspending agents, solubilizers, thickening agents, stabilizers, and preservatives.

The pharmaceutical composition can further comprise an excipient. Excipients that may be used include one or more carriers, surface active agents, thickening or emulsifying agents, solid binders, dispersion or suspension aids, solubilizers, colorants, flavoring agents, coatings, disintegrating agents, lubricants, sweeteners, preservatives, isotonic agents, and combinations thereof. The selection and use of suitable excipients is taught in Gennaro, ed., Remington: The Science and Practice of Pharmacy, 20th Ed. (Lippincott Williams & Wilkins 2003), the disclosure of which is incorporated herein by reference.

In some embodiments, the octadentate 3,4,3-LI-1,2-HOPO is highly effective for Pu, Np, Th, Am and Cf chelation *in vivo,* and its efficacy greatly exceeds that of the current actinide chelation standard CaNa₃-DTPA at low dosage. For example, the efficiency of 3,4,3-LI-1,2-HOPO for clearing circulating Pu from mouse tissues ranges from 100 times (skeleton) to 240 times (soft tissues) that of CaNa₃-DTPA in five different protocols. In addition, the optimal activity dose of 3,4,3-LI-1,2-HOPO for removing newly deposited Pu from mice is 2.5% of the dose of CaNa₃-DTPA used clinically. The tetradentate 5-LIO-Me-3,2-HOPO has potential therapeutic value for Pu, U, Am and Np, and its efficiency for clearing circulating Pu from mouse tissues ranges from 5 times (skeleton) to 15 times (liver) that of CaNa₃-DTPA.

**Oral Activity.** Both compounds are orally active actinide chelators: when administered orally to mice or beagles after a Pu injection, 3,4,3-LI-1,2-HOPO and 5-LIO-Me-3,2-HOPO can remove up to 80% and 60%, respectively, of the injected Pu. In addition, pharmacokinetic studies using ¹⁴C-labeled ligands show that both compounds are stable to metabolic degradation and are significantly more effective than CaNa₃-DTPA for removing newly deposited Pu, Np, Am and U from mice.

**Ligand Combination.** Octadentate 3,4,3-LI(1,2-HOPO), HOPO(1), is highly effective for *in vivo* chelation of Pu(IV) and Am(III). Tetradentate 5-LIO(Me-3,2-HOPO), HOPO (2), is structurally suitable for chelating Np(V) and U(VI).

### Applications of Chelating Agents for Particular MRI Contrast Agents

In some embodiments, one or more of the chelating agents provided herein can be used to reduce the risk associated with gadolinium-based contrast agents. In some embodiments, the chelator can be administered before, with, or after the use of a gadolinium-based contrast agent. Since their introduction into clinical practice in the United States in 1988, gadolinium-based contrast agents (GBCAs) have had a tremendous impact on magnetic resonance imaging (MRI). It is estimated that GBCAs are used in over one half of the 30 million MRI scans con- ducted each year in the United States. Contrast agents are considered to be safe with associated adverse event rates reported as 14.4 per 100,000 administrations globally. Only after 18 years of use were the first serious adverse effects reported, when nephrologists connected the administration of GBCAs to nephrogenic systemic fibrosis (NSF), prompting the U.S. Food and Drug Administration (FDA) to issue a warning in 2006. The exact pathophysiology of NSF remains unknown, but the dissociation of Gd ions from their chelating ligands has been accepted as the primary etiology, which is more likely to occur in patients with renal failure than in those with normal renal function because the excretion rate is reduced in the former, allowing time for the chelates to disassociate *in vivo.* Most cases of NSF reported in the literature have been associated with administration of nonionic, linear gadodiamide (Omniscan), though reports also described substantial incidents with another nonionic linear agent, gadoversetamide (OptiMARK), and with an ionic linear agent, gadopentetate dimeglumine (Magnevist). NSF has almost been completely eliminated since 2009 by effective screening of patients with renal disease and by avoiding GBCAs in patients with substantial renal disease or by utilizing more stable GBCA associated with extremely few or no cases of NSF. However, in the past 2 years, numerous studies regarding Gd deposition in neural tissues in patients with normal renal function have been published. This deposition was first postulated by MR imaging studies in which progressively increased signal intensity in the globi pallidi and/or dentate nuclei on unenhanced T1-weighted images in patients with normal renal function was related to multiple administrations of GBCAs. As with NSF, the agent most associated with this finding was gadodiamide, but it has also been shown with others, including gadopentetate dimeglumine.

Currently, there are 9 FDA-approved GBCAs, all of which are hydrophilic. They differ in their stability, ability to enhance proton relaxation rates, and distribution, with macrocyclic agents deemed to form more kinetically stable complexes. However, one of the most stable macrocyclic agents, gadobutrol (Gadavist), has also been shown to result in brain deposition. These findings suggest that all GBCAs should be evaluated individually, despite their molecular structures, and may all release some level of Gd. Gd, a lanthanide heavy metal, is highly toxic as a free ion and slowly excreted due to interactions with endogenous ligands and metal binding sites, especially those associated with calcium, zinc, and iron. If free Gd³⁺ is administered (in animal models), only 1% to 3% is eliminated per day with the remaining deposited predominantly in the liver, kidney, and bone. The free ion disrupts cellular processes, inhibits stretch-activated ion channels, and is one of the most efficient known calcium antagonists. Because of the large number of recent reports on Gd tissue deposition, the FDA is currently evaluating the risks of brain deposits and potential adverse effects and several clinical trials (ClinicalTrials.gov) are recruiting patients at the time of this application to evaluate the long term retention of Gd and associated adverse effects.

There is however, no viable option for the removal of internalized Gd. The only practical therapy to reduce the health consequences of Gd deposition would be treatment with chelating agents that form excretable complexes, although Gd, like other heavy metals, is among the most intractable elements to decorporate.

The mechanism of action of 3,4,3-LI(1,2-HOPO) is a chelation mechanism, in which the compound binds the targeted actinide and forms a stable complex that can be eliminated through excretion pathways. Chelation is likely to be clinically efficacious if the affinity of the chelating agent for the targeted actinide metal ion is higher than those of potential biological ligands (such as proteins and bone matrices) and if its affinity for the targeted actinide metal ion is more specific than for essential divalent metal ions. A quantitative tool to predict the efficacy of a chelating agent and confirm its potential for actinide chelation is the determination of the corresponding actinide complex stability constants *in vitro.* As outlined in Example 2 below, these constants were evaluated for metals such as Pu(IV) (Log *β*₁₁₀ > 41.5) or Cm(III) (Log *β*₁₁₀ = 21.8), and support the chelation mechanism of action by 3,4,3-LI(1,2-HOPO). The protonation and stability constants determined for Gd(III) (Log *β*₁₁₀ = 20.5) suggest that 3,4,3-LI(1,2-HOPO) forms some of the most stable Gd complexes known at physiological pH. This high affinity should translate into a high *in vivo* Gd removal rate, comparable to those observed with other elements such as Eu(III), Am(III), and Cm(III) in mouse models.

In some embodiments, chelation treatment with 3,4,3-LI(1,2-HOPO) can enhance the elimination long after Gd deposition. In some embodiments, this can enhance elimination of Gd deposition by 10, 20, 30, 40, 50, 100% or more.

In some embodiments, another way to diminish the potential health effects of Gd release from administered GBCAs is by preventing deposition.

3,4,3-LI(1,2-HOPO) prevents Gd deposition if administered once prior to, or at the same time as, a contrast agent. In some embodiments, 1, 2, 3, 4, 5 or more doses of the chelator can be administered prior to or overlapping with the administration of the Gd containing compound.

There are risks associated with the administration of GBCAs to patients with severely impaired kidney function (eGFR < 30). Patients with normal kidney function were initially not thought to be at risk of retaining Gd from GBCAs; however, some Gd from each dose of contrast may remain in the body of all patients exposed to GBCAs. The FDA, including its National Center for Toxicological Research (NCTR), and several academic and industrial research groups are actively working to understand the mechanism of Gd retention and to determine the extent of adverse health effects. There is however no current viable option for the removal of deposited Gd or the prevention of Gd deposition associated with GBCA administration. The development of a pre- and post-GBCA injection decorporation strategy is therefore an important research need that has the potential to shift the clinical MR imaging practice paradigms. Thus, in some embodiments, the chelators provided herein can be administered to subjects that have a risk of, or already have, severely impaired kidney function. This can be prophylactic therapy or reactive therapy.

3,4,3-LI(1,2-HOPO) has displayed up to 30 times more potency than DTPA at decorporating actinide ions in animal studies, and has the advantages of being orally available and extremely efficacious as a prophylactic treatment. Importantly, recent absorption, distribution, metabolism, and excretion studies performed with the ¹⁴C-labeled 3,4,3-LI(1,2-HOPO) have revealed that the ligand can penetrate the blood-brain barrier, as traces of ligand were found in the brain of rats several days after administration. This property is particularly relevant to target Gd deposited in the brain.

In some embodiments, any one or more of the chelators provided herein, such as 3,4,3-LI(1,2-HOPO) can be used in combination with a Gd based imaging agent (either before, with or after the administration of the imaging agent), so as to reduce the risk of Gd remaining in the host. The amount of 3,4,3-LI(1,2-HOPO) administered will be enough to reduce the level of remaining Gd to a desirable amount. In some embodiments, the amount of Gd remaining in the host is reduced by 1.1, 10, 20, 30, 40, 50, 60, 70, 80, 90, 95, 98, 99% or greater (e.g., 100%). The chelating agent can be administered so as to reduce the risk or symptoms, or duration of NSF or other disorder associated with the use of Gd based contrast agents in a subject. Metal poisoning can severely affect mental and physical development. Exposure to even low levels of heavy metals can cause damage over time, especially in children. A great risk is to brain development, where irreversible damage may occur. Higher levels can damage the kidneys and nervous system in both children and adults. In some embodiments, specific symptoms may include: 1) Pain - aching; burning, tingling, and/or prickling pain (paresthesia); deep bone pain; typically in extremities or joints, and sometimes in the location where the MRI occurred, like the head; 2) dermal changes - like tight skin, lesions, hyperpigmentation; most often in extremities; 3) muscle issues - twitching - small, local, rapid contractions and weakness; 4) ocular problems - worsening vision, dry eyes, bloodshot eyes; 5) cognitive symptoms; 6) ear, nose and throat - tinnitus, swallowing, and voice problems; 7) low body temperature; 8) hair loss; 9) itchy skin; 10) balance problems; 11) swelling of extremities (edema); and/or 12) a sense of an electrified, vibrating, twitching feeling typically just under the skin that is sometimes localized and at other times a more overall feeling.

In some embodiments, the chelating agent need not be administered to a subject, but can instead be used to purify one or more fluids (gas or liquid) so as to reduce an amount of heavy metal therein.

It is to be understood that this invention is not limited to particular embodiments described, as such may, of course, vary. It is also to be understood that the terminology used herein is for the purpose of describing particular embodiments only, and is not intended to be limiting, since the scope of the present invention will be limited only by the appended claims.

Where a range of values is provided, it is understood that each intervening value, to the tenth of the unit of the lower limit unless the context clearly dictates otherwise, between the upper and lower limits of that range is also specifically disclosed. Each smaller range between any stated value or intervening value in a stated range and any other stated or intervening value in that stated range is encompassed within the invention. The upper and lower limits of these smaller ranges may independently be included or excluded in the range, and each range where either, neither or both limits are included in the smaller ranges is also encompassed within the invention, subject to any specifically excluded limit in the stated range. Where the stated range includes one or both of the limits, ranges excluding either or both of those included limits are also included in the invention.

Unless defined otherwise, all technical and scientific terms used herein have the same meaning as commonly understood by one of ordinary skill in the art to which this invention belongs. Although any methods and materials similar or equivalent to those described herein can be used in the practice or testing of the present invention, the preferred methods and materials are now described. All publications mentioned herein are incorporated herein by reference to disclose and describe the methods and/or materials in connection with which the publications are cited.

It must be noted that as used herein and in the appended claims, the singular forms "a", "and", and "the" include plural referents unless the context clearly dictates otherwise. Thus, for example, reference to "a chelating agent" includes a plurality of such chelating agents, and so forth.

The invention having been described, the following examples are offered to illustrate the subject invention by way of illustration, not by way of limitation.

### EXAMPLE 1

Presented herein are two sets of studies aimed at investigating the administration time window of 3,4,3-LI(1,2-HOPO) and 5-LIO(Me-3,2-HOPO). Enhanced plutonium elimination is noted as early as 48 hours prior to contamination for prophylactic intraperitoneal or oral treatment, and as late as 7 days post-challenge for delayed intraperitoneal treatment. To ensure consistency among these and previously reported studies, contamination with soluble ²³⁸Pu-citrate was performed through a single intravenous injection, and the chosen animal model was the young adult female Swiss-Webster mouse. An advantage of these procedures is the need for only small amounts of radiological contaminant to obtain accurate counting statistics in tissue and excreta samples, avoiding large radionuclide inventories and reducing the amount of handled radioactive materials.

### 2. Materials and Methods

### 2.1 Contaminant and Ligand Solutions

A stock solution of ²³⁸Pu-nitrate in 4 M HNO₃ was purchased from Eckert and Ziegler Isotope Products (Valencia, CA, USA) and used to prepare injection solutions. Contamination doses consisted of 0.2 mL aliquots of solutions containing 0.74 kBq (1.16 ng) of ²³⁸Pu in 0.008 M sodium citrate and 0.14 M NaCl, pH 4. The ligands 3,4,3-LI(1,2-HOPO) and 5-LIO(Me-3,2-HOPO) were prepared by Synthetech, Inc. (Albany, OR, USA) and Albany Molecular Research, Inc. (Albany, NY, USA), respectively, as described previously [19]. DTPA was obtained from Sigma-Aldrich (St. Louis, MO, USA) and was formulated as Ca-DTPA using CaCO₃ and NaOH, similarly to the formerly available drug product commercialized by Hameln Pharmaceuticals gmbh (Hameln, Germany). Ligand solutions were prepared such that the selected dosages (30 µmol/kg for Ca-DTPA, 30 or 100 µmol/kg for 3,4,3-LI(1,2-HOPO), and 100 or 200 µmol/kg for 5-LIO(Me-3,2-HOPO)) were contained in 0.5 mL of 0.14 M NaCl, with the pH adjusted to 7.4-8.4 with 1 N NaOH. All solutions were filter-sterilized (0.22 µm) prior to administration. The concentration of each solution was verified by high-performance liquid chromatography, following modified published methods.

### 2.2 Animals and General Procedures

All procedures and protocols used in the described *in vivo* studies were reviewed and approved by the Institutional Animal Care and Use Committee of Lawrence Berkeley National Laboratory and were performed in AAALAC accredited facilities. The animals used were young adult (86 ± 6 days old for delayed treatment experiment and 90 ± 3 days old for prophylactic treatment experiment) female (30.7 ± 4.0 g for delayed treatment experiment and 30.8 ± 1.6 g for prophylactic treatment experiment) Swiss-Webster mice (Simonsen Laboratories, Gilroy, CA, USA). Gross body and tissue compositions, plasma, extracellular fluid, and red cell volumes of the whole body, major tissues and organs of these mice (intact or bled 25 to 40% of their total blood volume) have been determined previously. Mice were kept under a 12-hour light cycle with controlled temperature (18-22°C) and relative humidity (30-70%), and were given water and food *ad libitum.* Each group of mice was housed together in a plastic stock cage lined with a 0.5 cm layer of highly absorbent low-ash pelleted cellulose bedding (ALPHA-dri^{®}) for separation of urine and feces. Intravenous (iv) injections into a warmed lateral tail vein, intraperitoneal (ip) injections, oral administrations (po, through gastric intubation) and euthanasia were performed under isoflurane anesthesia. Treatment dose volumes were adjusted based on the weight of the mouse, with a 0.5 mL volume corresponding to a 35 g mouse. To probe the effect of delayed treatment, groups of five mice were injected iv with a single dose of ²³⁸Pu-citrate, and ligand or control saline solutions were administered ip once at the following post-contamination treatment times: 1 h, 5 h, 16 h, 24 h, 3 d, 7 d. Excreta were collected daily for 7 days. Animals were euthanized 7 days after treatment. To probe the effect of prophylactic treatment, groups of five mice were first administered ligand or control saline solutions ip or po once at the following pre-contamination treatment times: -1 h, -6 h, -16 h, -24 h, -30 h, - 40 h, -48 h. Mice were then injected iv with a single dose of ²³⁸Pu-citrate and excreta were collected daily for 3 days. Animals were euthanized 3 days (72 h) after contamination. Mice were euthanized by cervical dislocation over their respective cage to collect the urine expelled at death, and immediately wrapped in plastic and frozen for later dissection.

### Tissue Sampling and Processing

After partial thawing of the frozen mice, livers and kidneys were dissected, and the abdominal tissue remainder (ATR, which includes intact gastrointestinal (GI) tract, reproductive organs, spleen, urinary bladder, and abdominal fat) was removed. The livers, kidneys, ATR, and partially eviscerated carcasses were managed as individual samples. Feces samples were separated manually from urine-stained cellulose bedding and treated as group samples (one group per cage). All samples were dried at 100°C and dry ashed at 575°C. The ashed samples were treated with concentrated HNO₃. These acidified solutions were then homogenized in dilute HNO₃ and mixed with Ultima Gold (Perkin Elmer, Shelton, CT, USA) for detection of radionuclides by liquid scintillation counting (Packard Tri-Carb model B4430, Perkin Elmer).

### Data Management and Analysis

All experiments used radioactive ²³⁸Pu as a contaminant and were therefore managed as metabolic balance studies, in which all tissues and excreta were radioanalyzed; average radiochemical recoveries were all greater than 95% injected dose (ID). The experimental data are reported as radionuclide fractions, expressed as percent of injected ²³⁸Pu (%ID), and values are arithmetic means ± SD. When comparing values between groups in the decorporation studies, the term "significant" is used in the statistical sense, indicating p < 0.01 by one-way analysis of variance (ANOVA) followed by adequate post-hoc analysis. The Dunnett's multiple-comparison test was used to compare groups of animals treated with a chelating agent to the corresponding control group that was administered saline, while the Tukey's Honestly Significant Difference (HSD) multiple-comparison test was used to perform pairwise comparisons between all groups treated with a chelating agent. Both tests were set at the 99% confidence interval level. All statistical analyses were performed using GraphPad Prism 5 (GraphPad Software, Inc., San Diego, CA, USA).

### Results

The plutonium elimination enhancement promoted by a single parenteral or oral administration with one of the experimental decorporation agents 3,4,3-LI(1,2-HOPO) and 5-LIO(Me-3,2-HOPO) was probed in young adult female Swiss-Webster mice, dependent on the time of treatment administration. In the first study, chelation treatment was administered parenterally once after the challenge event, at times varying from 1 hour to 7 days post-contamination, and mice were euthanized 7 days after treatment. In the second study, a single treatment was administered prophylactically either parenterally or orally at times varying from 48 hours to 1 hour prior to contamination, and mice were euthanized 3 days after the contamination event. In both studies, the primary efficacy endpoint was radionuclide body content reduction at the time of euthanasia, compared to the saline-treated control groups. For parenteral treatment cases, comparisons could also be drawn with DTPA-treated groups. The *in vivo* portions of both studies were accomplished without incident. As expected from previously reported results, dose levels for the experimental ligands 3,4,3-LI(1,2-HOPO) and 5-LIO(Me-3,2-HOPO) did not result in any discernable adverse effects.

### Delayed Parenteral Chelation Treatment

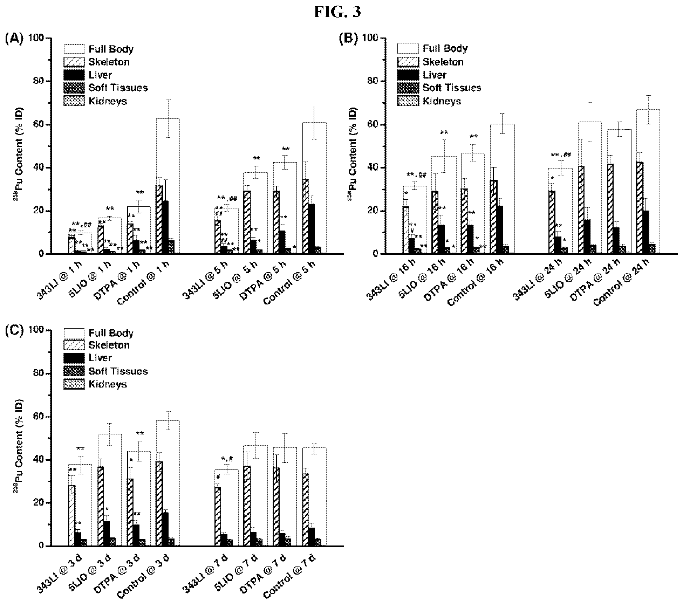

For the study where mice were treated between 1 hour and seven days after contamination, and euthanized seven days after treatment, total ²³⁸Pu body content and distribution results at the different necropsy times were subjected to statistical analysis and are depicted in Figure **3****.** At treatment time points ranging from 1 hour to 16 hours post-contamination, a single parenteral dose of either 3,4,3-LI(1,2-HOPO) or 5-LIO(Me-3,2-HOPO) resulted in significant increases in ²³⁸Pu elimination rates and total body burden and distinct tissue content reductions, as compared to saline-treated controls. However, only groups chelated with 3,4,3-LI(1,2-HOPO) at those time points lead to decreases in ²³⁸Pu content that were significantly better than those observed after DTPA treatment. In addition, 3,4,3-LI(1,2-HOPO) was the only chelating option for which ²³⁸Pu elimination was significantly enhanced when treatment was administered at delayed time points from 24 hours to 7 days after contamination. It was also the only ligand that resulted in significantly reduced ²³⁸Pu skeleton content at all tested treatment time points. The decorporation efficacy decreased with increasing delays in treatment.

FIG. 3 depicts the total ²³⁸Pu body content and distribution at 7 days after a single time-delayed ip chelation treatment. Young adult female Swiss-Webster mice injected iv with ²³⁸Pu-citrate; saline or treatment (3,4,3-LI(1,2-HOPO) [30 µmol/kg], 5-LIO(Me-3,2-HOPO) [100 µmol/kg], or Ca-DTPA [30 µmol/kg]) administered ip at 1 h, 5 h **(A),** 16 h, 24 h **(B),** 3 d, or 7 d **(C)** post-contamination; mice euthanized 7 days after treatment. Data is expressed as percent of injected ²³⁸Pu dose (% ID, mean ± SD) for each five-mouse group. Groups with significantly lower retention than for control mice are indicated by * or ** (p < 0.05 or p < 0.01, 1-way ANOVA with post hoc Dunnett's multiple comparison test), while groups with significantly lower retention than for Ca-DTPA-treated mice are indicated by ^{#} or ^{##} (p < 0.05 or p < 0.01, 1-way ANOVA with post hoc Tukey's HSD multiple comparison test).

Nevertheless, the elimination rates followed different kinetics depending on the time elapsed between contamination and treatment, as shown in FIG. 4. While prompt chelation treatment (1 hour post-contamination) with HOPO ligands promoted the immediate excretion of more than 80% of the injected dose, the rates of elimination became slower than those observed in control animals by 2 or 3 days post-treatment for 5-LIO(Me-3,2-HOPO) or 3,4,3-LI(1,2-HOPO), respectively. The excretion enhancements promoted by delayed treatment (7 days post-contamination) were not as pronounced; however, the rates of elimination observed in the 7 days following treatment with either HOPO ligand were still comparable or faster than those observed in control animals. While 3,4,3-LI(1,2-HOPO) exhibited largely better efficacies than 5-LIO(Me-3,2-HOPO) at all time points, the rates of elimination were also notably different between both ligands: beyond the initial immediate action of the ligands observed within 24 hours after treatment, 5-LIO(Me-3,2-HOPO) did not display significant sustained action in contrast with 3,4,3-LI(1,2-HOPO) or DTPA. The cumulative excretion patterns shown in FIG. 4 provide a clear visual representation of these differences. For treatment time points between 16 hours and 7 days, the sustained action of DTPA was visible over several days after treatment, with a curve-shaped excretion pattern, reaching similar (even higher in one case) excretion levels as with 5-LIO(Me-3,2-HOPO) by the fourth day post-treatment. Similarly, the prolonged efficacy of 3,4,3-LI(1,2-HOPO) was visible throughout the seven-day excreta collection period of this study, revealing 3,4,3-LI(1,2-HOPO) to be not only the most efficacious, but also the fastest treatment option to reduce ²³⁸Pu contamination.

Daily fecal and urinary excretion rates for each chelating treatment were also examined and are displayed in FIG. 5. These panels show that independently of the post-contamination treatment time, both 3,4,3-LI(1,2-HOPO) and 5-LIO(Me-3,2-HOPO) systematically enhanced excretion primarily through the biliary pathway, with only a minor urinary component, unlike DTPA treatment. Daily ²³⁸Pu fecal (left panels **A, C, E, G)** and urinary (right panels **B, D, F**, **H**) output after a single 3,4,3-LI(1,2-HOPO) **(A** and **B),** 5-LIO(Me-3,2-HOPO) (**C** and **D**) , DTPA **(E** and **F),** or saline **(G** and **H),** time-delayed ip chelation treatment at 1 h, 5 h, 16 h, 24 h, 3 d, or 7 d post-contamination. Young adult female Swiss-Webster mice injected iv with ²³⁸Pu-citrate; saline or treatment (3,4,3-LI(1,2-HOPO) [30 µmol/kg], 5-LIO(Me-3,2-HOPO) [100 µmol/kg], or Ca-DTPA [30 µmol/kg]) administered ip at 1 h, 5 h, 16 h, 24 h, 3 d, or 7 d post-contamination; mice euthanized 7 days after treatment. Excreta of each five-mouse group were pooled daily and standard deviations are not available as data are calculated from the group-collected excretion.

### 3.2 Prophylactic Parenteral or Oral Chelation Treatment

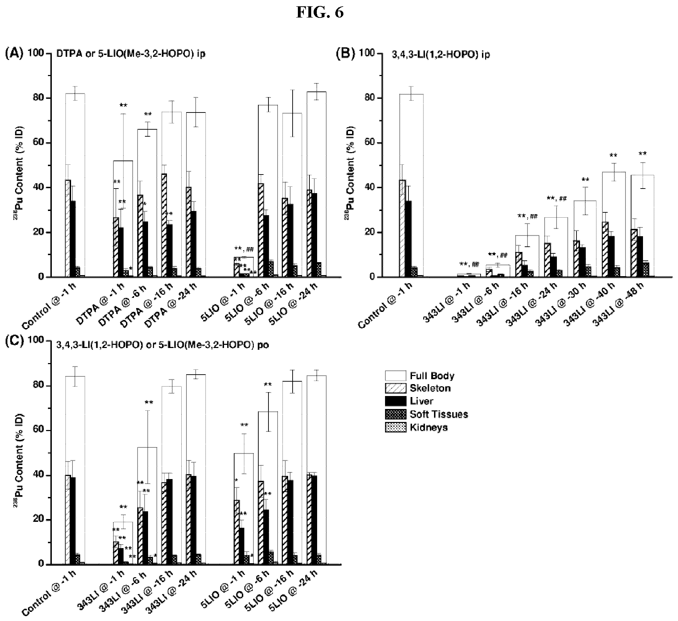

For this study where mice were treated prophylactically between 1 hour and 48 hours prior to contamination, total ²³⁸Pu body content and distribution results at three days post-contamination were subjected to statistical analysis and are depicted in FIG. 6. Parenteral administrations of 5-LIO(Me-3,2-HOPO) or DTPA were effective at significantly reducing body and tissue ²³⁸Pu burden over a very short prophylactic window (1 hour and 6 hours, respectively), as shown in FIG. 6, Panel A. In contrast, 3,4,3-LI(1,2-HOPO) exhibited remarkable prophylactic activity even when injected once 48 hours prior to contamination (FIG. 6, Panel **B).** All animals showed large significant reductions in full body, skeleton, liver, soft tissue, and kidney content of ²³⁸Pu for the groups treated parenterally with 3,4,3-LI(1,2-HOPO), compared to the corresponding saline- and DTPA-treated groups. As expected, decorporation efficacy decreased with increasing delays between treatment and contamination. Nevertheless, the efficacy level observed after a 3,4,3-LI(1,2-HOPO) injection 48 hours before contamination (up to 50% ²³⁸Pu excreted within 3 days) also suggests that the prophylactic window could be extended further. The prophylactic efficacy of 3,4,3-LI(1,2-HOPO) and 5-LIO(Me-3,2-HOPO) was also probed after oral administration (FIG. 6, Panel **C).** Groups treated with either ligand in the 6-hour prophylactic window showed significant reductions in total body, skeleton and liver content of ²³⁸Pu compared to the saline control groups. However, the low oral bioavailability of 3,4,3-LI(1,2-HOPO) was reflected in the lack of decorporation efficacy for earlier treatments.

As noted above, FIG. 6 depicts total ²³⁸Pu body content and distribution 3 days after a contamination event preceded by a single prophylactic chelation treatment. Young adult female Swiss-Webster mice injected iv with ²³⁸Pu-citrate; saline or treatment (3,4,3-LI(1,2-HOPO) [30 µmol/kg ip or 100 µmol/kg po], 5-LIO(Me-3,2-HOPO) [100 µmol/kg ip or 200 µmol/kg po], or Ca-DTPA [30 µmol/kg ip]) administered ip **(A** for 5-LIO(Me-3,2-HOPO) and DTPA, and **B** for 3,4,3-LI(1,2-HOPO)) or po **(C)** at 1 h, 6 h, 16 h, 24 h, 30 h, 40 h, or 48 h prior to contamination; mice euthanized 3 days after contamination. Data was expressed as percent of injected ²³⁸Pu dose (% ID, mean ± SD) for each five-mouse group. Groups with significantly lower retention than for control mice are indicated by * or ** (p < 0.05 or p < 0.01, 1-way ANOVA with post hoc Dunnett's multiple comparison test), while groups with significantly lower retention than for Ca-DTPA-treated mice are indicated by ^{#} or ^{##} (p < 0.05 or p < 0.01, 1-way ANOVA with post hoc Tukey's HSD multiple comparison test). In Panel **B,** all distinct tissue content bars should indicate **^{, ##} (-1 h to -24 h) or ** (-30 h to -48 h), but symbols were omitted for clarity.

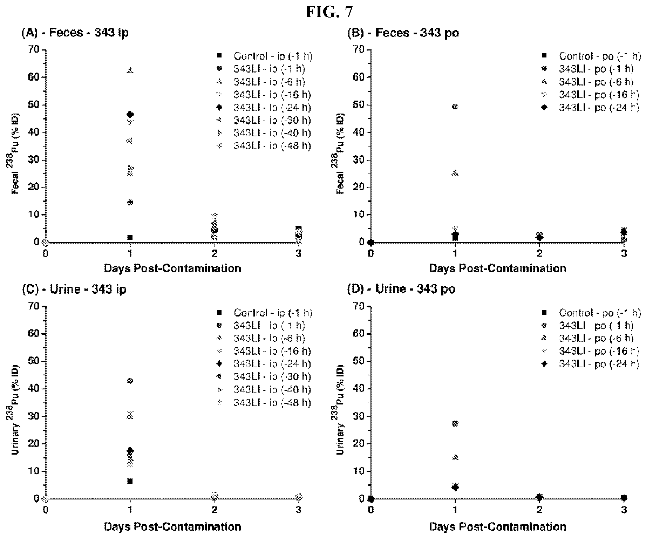

Finally, daily fecal and urinary outputs after parenteral or oral 3,4,3-LI(1,2-HOPO) prophylactic administration are displayed in FIG. 7. All panels show a rapid loss of elimination enhancement beyond the first day after contamination. For all treatment regimens but one, ²³⁸Pu excretion is again predominantly fecal. However, in the case of parenteral treatment with 3,4,3-LI(1,2-HOPO) right before contamination, at the -1 hour time point, the ²³⁸Pu level detected in the urine was 3-fold higher than the fecal output. For the results in FIG. 7, young adult female Swiss-Webster mice injected iv with ²³⁸Pu-citrate; 3,4,3-LI(1,2-HOPO) [30 µmol/kg ip or 100 µmol/kg po] administered ip or po at 1 h, 6 h, 16 h, 24 h, 30 h, 40 h, or 48 h prior to contamination; mice euthanized 3 days after contamination. Excreta of each five-mouse group were pooled daily and standard deviations are not available as the data are calculated from the group-collected excretion.

### EXAMPLE 2

Recent studies in humans and animals aimed at determining the level of Gd released from GBCAs were mostly performed using MR imaging as the method of choice for Gd detection. While MRI is a non-invasive technique, enhanced resolution is mostly achieved when Gd is chelated, which reduced hydrogen-proton relaxation times, limiting the detection sensitivity for free Gd. Scarce older studies have described the biodistribution of radiolabeled GBCAs in mice and rats, ameliorating measurement accuracy and demonstrating the larger-than-anticipated extend of Gd release. However, these studies used the low-energy gamma-emitter ¹⁵³Gd isotope, detected through gamma-counting. Here, one will use the alpha-emitter ¹⁴⁸Gd, which would then be detected by liquid scintillation counting, according to published protocols, a method that offers much higher sensitivity. In addition, the combination of radiotracer determination with MR measurement will assure that we can discriminate free from chelated Gd *in vivo.* Finally, access to the high-affinity chelator 3,4,3-LI(1,2-HOPO) offers decorporation prospects unparalleled with any other existing and available ligand.

Controlled non-clinical studies will be conducted in established mouse models injected with free and chelated ¹⁴⁸Gd. The goal of these studies is to determine the *in vivo* removal of Gd by 3,4,3-LI(1,2-HOPO) and to determine the impact of prophylactic 3,4,3-LI(1,2-HOPO) on the GBCA MR image enhancement. For each of the following studies, excreta and several tissues will be collected and analyzed to determine metal levels. Most of the details of the technical methods to be used have been published, including the ligand solutions, animals, animal injection procedures, autopsy procedures, collection of excreta, preparation of samples, radioanalysis and data management. Baseline studies including animal control groups will also be performed. A series of studies will probe the potency of 3,4,3-LI(1,2-HOPO) to increase the rates of elimination of ¹⁴⁸Gd from Swiss-Webster mice pre- or post-Gd administration under different chemical forms: in a citrate solution to mimic the free ion, or chelated with either the linear DTPA-BMA (gadodiamide) or the macrocyclic DO3A (gadoterate) ligands. MR images will also be acquired to probe the effect of 3,4,3-LI(1,2-HOPO) treatment on the efficiency of the injected GBCAs. Detailed experimental procedures are described below.

Test system: Young adult Swiss-Webster mice. A large majority of radionuclide decorporation efficacy studies previously performed with 3,4,3-LI(1,2-HOPO) were conducted on laboratory mice. Mice are commonly used for metabolic and toxicity studies, because they are appropriate small- scale acute models for larger mammals. Mice were used in part because Pu metabolism and chelate action had already been studied in that animal, but there were other factors to consider as well. The animal chosen for the primary investigations was the young adult female Swiss-Webster mouse, an outbred strain of stable size and docile behavior. The mice were used at 11-15 weeks of age and 30 ± 3 g body weight. At that age, the mouse skeleton is nearly mature, and the long bones have attained 98% of their maximum length. An animal model with a mature skeleton more closely resembles a human adult with respect to the degree and extent of bone remodeling. These are important considerations in interpreting the results of chelation therapy, as they will significantly affect the metal biokinetics and deposition kinetics in sites such as the skeleton. The last important advantage, unique to the study of metal chelators using radiotracers, is the generation of much smaller amounts of radioactively contaminated wastes.

Test challenge: ¹⁴⁸Gd complexed to citrate, DTPA-BMA (gadodiamide), or DO3A (gadoterate). The Gd³⁺ ion may exhibit different biokinetics, depending on its chemical form. Parameters and variables of consideration in efficacy studies must therefore include adequate control and comparison groups. Gadodiamide and gadoterate will be used as the reference GBCAs, since DTPA-BMA is a linear agent that exhibits one of the lowest Gd complex stability constants and has resulted in most of the observed brain deposition, and DO3A is one of the most stable macrocyclic chelators. Metal solutions will be formulated according to published protocols to mimic clinically relevant GBCA dose levels, and administered to female young-adult Swiss-Webster mice by intravenous injections. Systematic injections of soluble radiotracers have been shown to contaminate animals with a highly reproducible isotopic burden, which is important to assess the efficacy of a chelating agent in an accurate manner. ¹⁴⁸Gd will be obtained commercially from the U.S. DOE's National Isotope Development Center. All ligands and contrast agents are commercially available. Table 1 summarizes the study design.

Treatment regimen. Treatment with the chelator 3,4,3-LI(1,2-HOPO) will be administered parenterally (intraperitoneal - ip - injection) or orally (po, gastric intubation) at 8 time points ranging from 24 to 1 hour before or after Gd contamination. Both parenteral and oral treatment regimen have been optimized for the decorporation of actinides in multiple dosing regimen starting 24 hours after metal contamination. The chosen parenteral and oral dose levels (100 and 600 µmol/kg, respectively) are based on previous optimization studies using this particular mouse model. For each ¹⁴⁸Gd form study, 72 female and 72 male animals will be randomly assigned to one of 18 treatment groups (4 mice per group, statistical justification provided in Vertebrate Animal Use section), as summarized in Table 1. Mice will be group-housed in disposable stock cages lined with absorbent low-ash pelleted cellulose bedding to facilitate the separation of urine and feces. All animals in these studies will be monitored for adverse health effects and euthanized 72 hours post-Gd injection. Urine and feces will be collected daily from contamination to necropsy. Full necropsies will be conducted and all samples collected for heat and chemical treatments, and subsequent analysis by liquid scintillation counting.

Endpoints and success criteria. Efficacy can be based on direct measurement of the elimination of the radiotracer through feces and/or urine at various time points after administration of the decorporation agent. Reduction in metal content following administration of the chelating agent in an animal efficacy study will translate into general prevention of Gd toxicity. In these prophylactic efficacy studies, metal decorporation efficacy will be assessed based on direct measurement of the elimination of the radiotracer at a single time point, 72 hours after contamination. Successful decorporation will be characterized by significant decrease in radiotracer body content and increase in bodily excretion as compared to control groups, which include contaminated and vehicle-treated animals. MR Imaging will be performed for groups treated with prophylactic 3,4,3-LI(1,2-HOPO) in the 4-h time range after Gd injection (to probe the effect of 3,4,3-LI(1,2-HOPO) on MR images) as well as right before necropsy (to correlate MR images with radionuclide distribution results).

These studies will therefore provide clear evidence of the potential decorporation efficacy of 3,4,3-LI(1,2-HOPO) for Gd. The indication of 3,4,3-LI(1,2-HOPO) as a decorporation agent will be enlarged to include Gd.

| | | | | | |
|---|---|---|---|---|---|
| | | | | | |

| **Group** | **Treatment** | **Dose (µmol/kg)^{c}** | **# ofAnimal s** | **Treatment Route** | **Dosing Schedule** |
|---|---|---|---|---|---|
| 1A-1C^{d} | Vehicle Control | NA | 4F/4M | ip | once at 24 h pre-contamination |
| 2A-2C^{d} | 3,4,3-LI(1,2-HOPO) | 100 | 4F/4M | ip | once at 24 h pre-contamination |
| 3A-3C^{d} | 3,4,3-LI(1,2-HOPO) | 100 | 4F/4M | ip | once at 12 h pre-contamination |
| 4A-4C^{d} | 3,4,3-LI(1,2-HOPO) | 100 | 4F/4M | ip | once at 6 h pre-contamination |
| 5A-5C^{d} | 3,4,3-LI(1,2-HOPO) | 100 | 4F/4M | ip | once at 1 h pre-contamination |
| 6A-6C^{d} | 3,4,3-LI(1,2-HOPO) | 100 | 4F/4M | ip | once at 1 h post-contamination |
| 7A-7C | 3,4,3-LI(1,2-HOPO) | 100 | 4F/4M | ip | once at 6 h post-contamination |
| 8A-8C | 3,4,3-LI(1,2-HOPO) | 100 | 4F/4M | ip | once at 12 h post-contamination |
| 9A-9C | 3,4,3-LI(1,2-HOPO) | 100 | 4F/4M | ip | once at 24 h post-contamination |
| 10A-10C^{d} | Vehicle Control | NA | 4F/4M | po | once at 24 h pre-contamination |
| 11A-11C^{d} | 3,4,3-LI(1,2-HOPO) | 600 | 4F/4M | po | once at 24 h pre-contamination |
| 12A-12C^{d} | 3,4,3-LI(1,2-HOPO) | 600 | 4F/4M | po | once at 12 h pre-contamination |
| 13A-13C^{d} | 3,4,3-LI(1,2-HOPO) | 600 | 4F/4M | po | once at 6 h pre-contamination |
| 14A-14C^{d} | 3,4,3-LI(1,2-HOPO) | 600 | 4F/4M | po | once at 1 h pre-contamination |
| 15A-15C^{d} | 3,4,3-LI(1,2-HOPO) | 600 | 4F/4M | po | once at 1 h post-contamination |
| 16A-16C | 3,4,3-LI(1,2-HOPO) | 600 | 4F/4M | po | once at 6 h post-contamination |
| 17A-17C | 3,4,3-LI(1,2-HOPO) | 600 | 4F/4M | po | once at 12 h post-contamination |
| 18A-18C | 3,4,3-LI(1,2-HOPO) | 600 | 4F/4M | po | once at 24 h post-contamination |
| **Total: 216F/216M** | | | | | |
| **72F/72M mice per Gd form (¹⁴⁸Gd-citrate groups "nA", ¹⁴⁸Gd-gadodiamide groups "nB", ¹⁴⁸Gd-gadoterate groups "nC")** | | | | | |

| | | | | | |
|---|---|---|---|---|---|
| ^{*a*148}Gd contamination is achieved by intravenous injection in a warmed lateral tail vein of 0.2 mL of the challenge form (citrate, diamide, doterate). Treatments and control vehicle are administered by intraperitoneal injection or oral gavage. *^{b}*Whole animal, blood, and tissue challenge isotope content are determined at one unique time point: 72 hours post metal challenge. Excreta are collected daily post contamination until necropsy. *^{c}*Based on the results from previous dose optimization studies, with a target volume of administration of 0.5 mL for a 35 g mouse. *^{d}*MR images will be acquired for those groups between 1 and 4 h post-Gd administration and shortly prior to necropsy on a 1.5T Siemens Avanto, using a wrist extremity coil, according to published methods (48). | | | | | |

### EXAMPLE 3

A subject that is to receive a MRI contrast agent that includes Gd is identified. The subject has severely impaired kidney function. The subject is administered an effective, prophylactic amount of 3,4,3-LI(1,2-HOPO) (between 1 and 1000 microMole/Kg), and then administered an amount of the Gd based MRI contrast agent. The amount of the Gd contrast agent in the subject two days after the MRI will be lower than if the subject had not received the chelating agent.

### EXAMPLE 4

A subject that is to receive a MRI constrast agent that includes Gd is identified. The subject is administered the Gd based MRI contrast agent. Within 7 days of this, an effective amount of 3,4,3-LI(1,2-HOPO) is administered to the subject. The amount of the Gd contrast agent in the subject over the next period of days will be lower than if the subject had not received the chelating agent.

### EXAMPLE 5

A subject that is to be in an environment where exposure to a heavy metal including one of lead, tin, cadmium, scandium, and yttrium, is likely is identified. The subject is administered an effective, prophylactic amount of 3,4,3-LI(1,2-HOPO) (between 1 and 1000 microMole/Kg). The subject can then face exposure to the heavy metal within the next two days, with an enhanced ability to have the heavy metal chelated and then excreted from the subject.

### EXAMPLE 6

A subject that has been exposed to gadolinium, lead, tin, and/or cadmium is identified. The subject is administered an effective, amount of 3,4,3-LI(1,2-HOPO) (between 1 and 1000 microMole/Kg). The amount of the gadolinium, lead, tin, and/or cadmium in the subject over the next period of days will be lower than if the subject had not received the chelating agent. In the alternative, the administration of the 3,4,3-LI(1,2-HOPO) can take place 1, 2, 3, 4, 5, 6, or 7 days after the subject has been exposed to gadolinium, lead, tin, and/or cadmium.

### EXAMPLE 7

A subject that has been chronically exposed to lead from environmental contamination is identified. The subject is administered an effective, amount of 3,4,3-LI(1,2-HOPO) (between 1 and 1000 microMole/Kg). The chelating agent is repeatedly administered, if needed. The amount of lead in the subject over the next period of days will be lower than if the subject had not received the chelating agent.

### EXAMPLE 8

A subject that has received one or more MRI scans in the past, employing Gd, is identified. The subject is administered an effective, amount of 3,4,3-LI(1,2-HOPO) (between 1 and 1000 microMole/Kg), in one or more doses. The amount of Gd in the subject over the next period of days will be lower than if the subject had not received the chelating agent.

### EXAMPLE 9

The present example is an evaluation of the metal-removal effectiveness of various molecules, including 3,4,3-LI(1,2-HOPO) and DTPA. The arrangements tested (which compounds, how much, how long pre or post-contamination the compound was administered, etc.) are outlined in Table 9. 1.

| | | | | | |
|---|---|---|---|---|---|
| | | | | | |

| **Group** | **Treatment** | **Dose (µmol/kg)^{c}** | **# of Animals** | **Treatment Route** | **Dosing Schedule (Single Dose)** |
|---|---|---|---|---|---|
| A | Vehicle Control | NA | 4F | ip | 24 h pre-contamination |
| B | 3,4,3-LI(1,2-HOPO) | 100 | 4F | ip | 24 h pre-contamination |
| C | 3,4,3-LI(1,2-HOPO) | 100 | 4F | ip | 1 h pre-contamination |
| D | DTPA | 30 | 4F | ip | 1 h pre-contamination |
| E | 3,4,3-LI(1,2-HOPO) | 100 | 4F | ip | 1 h post-contamination |
| F | DTPA | 100 | 4F | ip | 1 h post-contamination |
| G | 3,4,3-LI(1,2-HOPO) | 100 | 4F | ip | 24 h post-contamination |
| H | 3,4,3-LI(1,2-HOPO) | 100 | 4F | ip | 48 h post-contamination |
| **Total: 32F** | | | | | |

| | | | | | |
|---|---|---|---|---|---|
| ^{*a*153}Gd contamination achieved by iv injection in a warmed lateral tail vein of 0.2 mL of the challenge form (citrate). Treatments and control vehicle administered by ip injection. *^{b}*Whole animal and tissue challenge isotope content determined at one unique time point: 4 days post metal challenge. Excreta collected daily post contamination until necropsy. | | | | | |

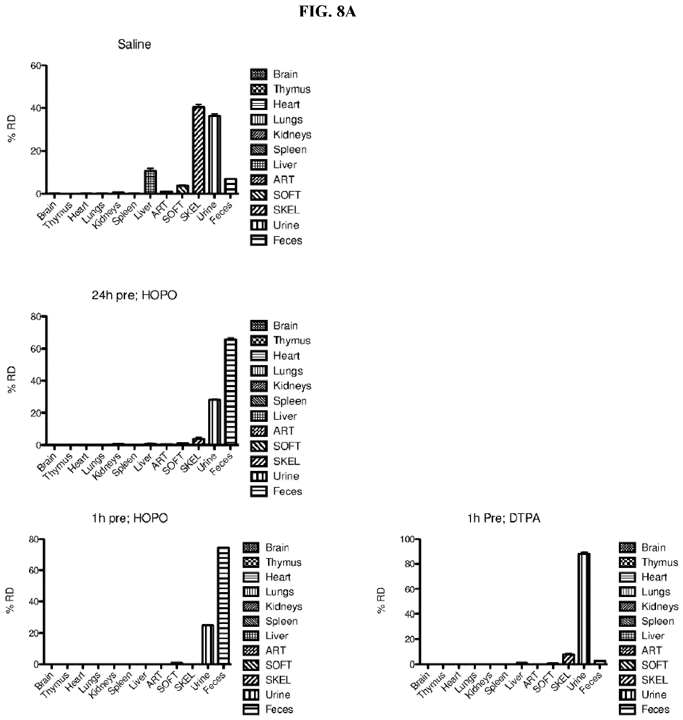

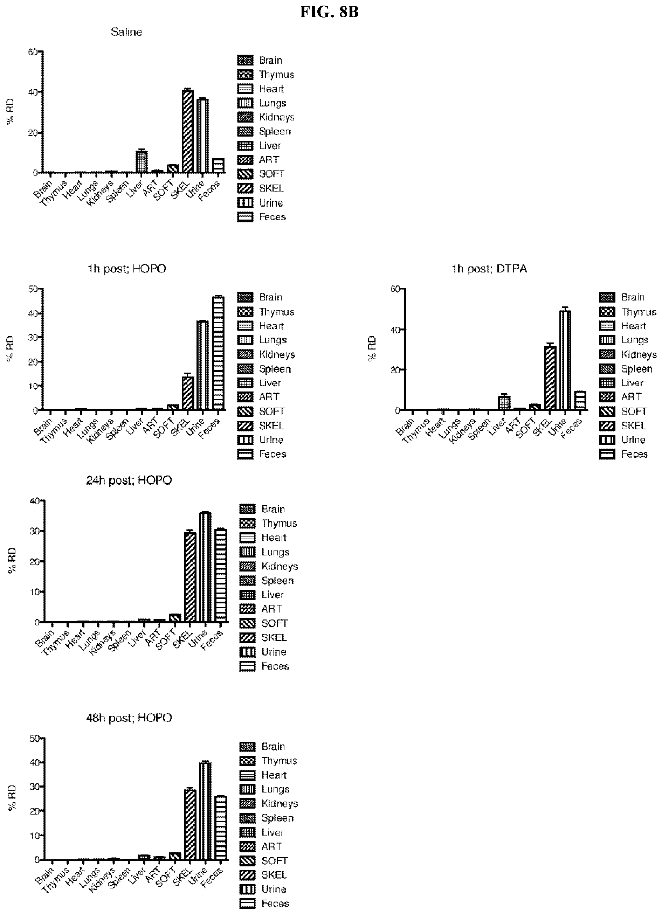

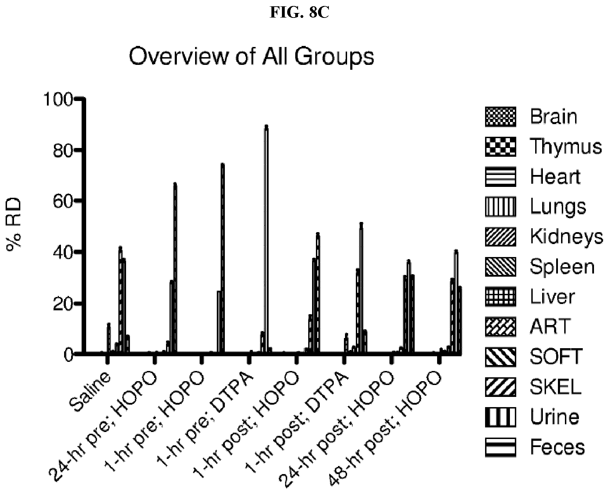

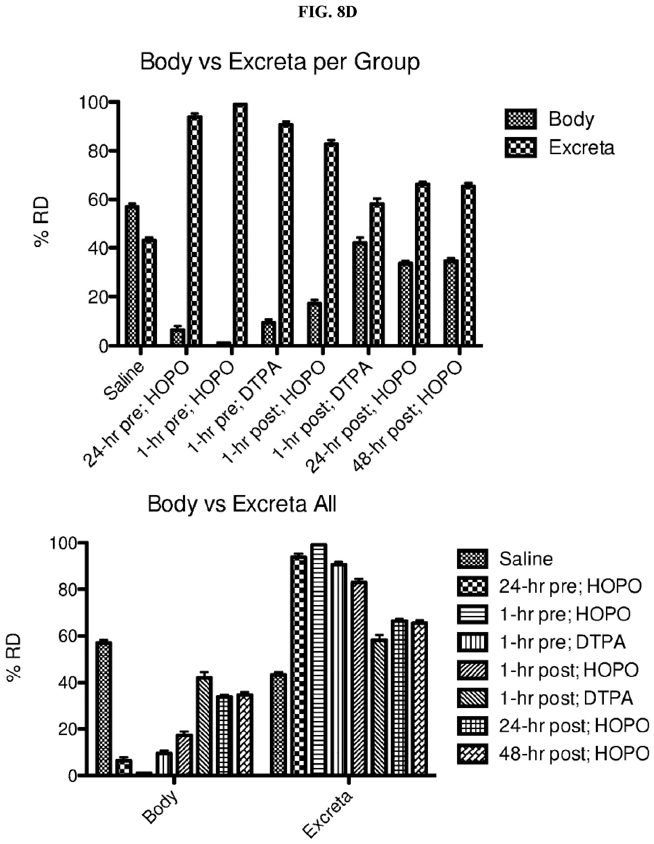

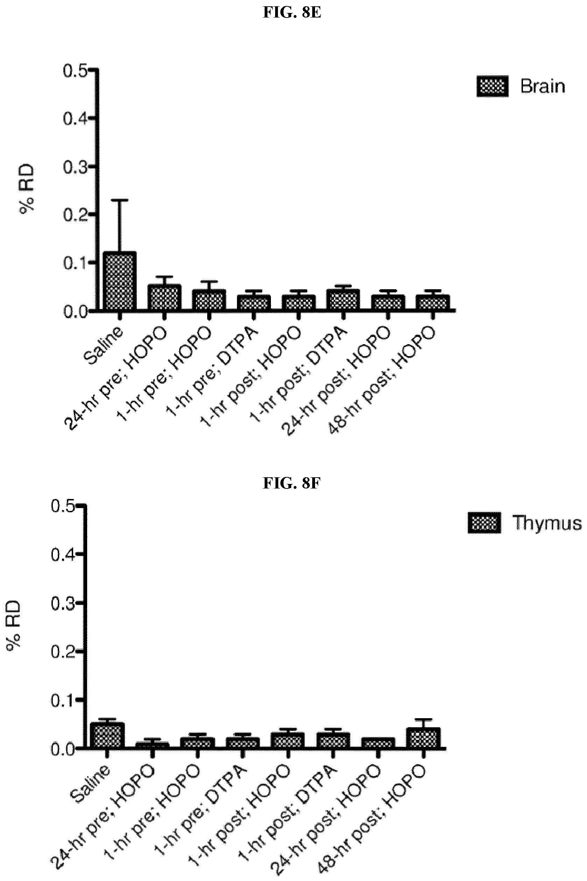

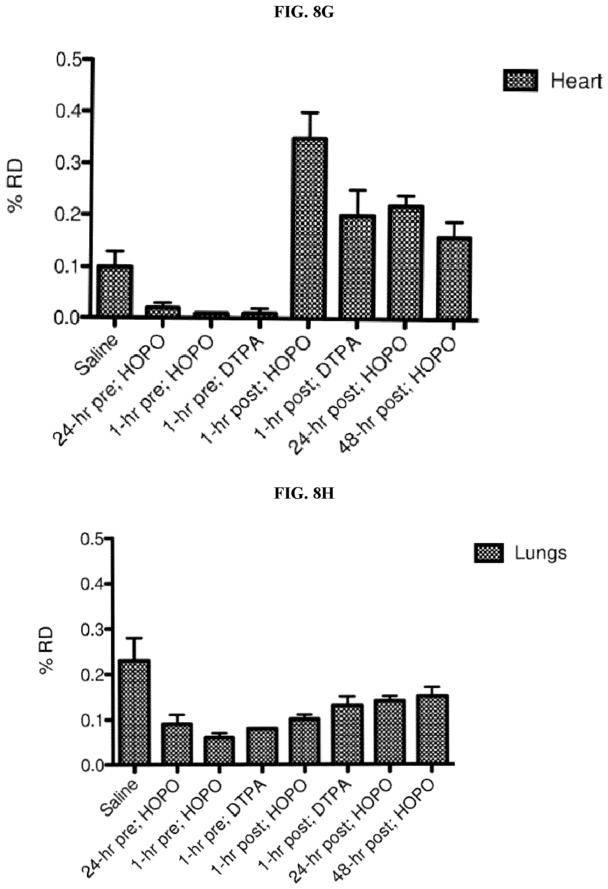

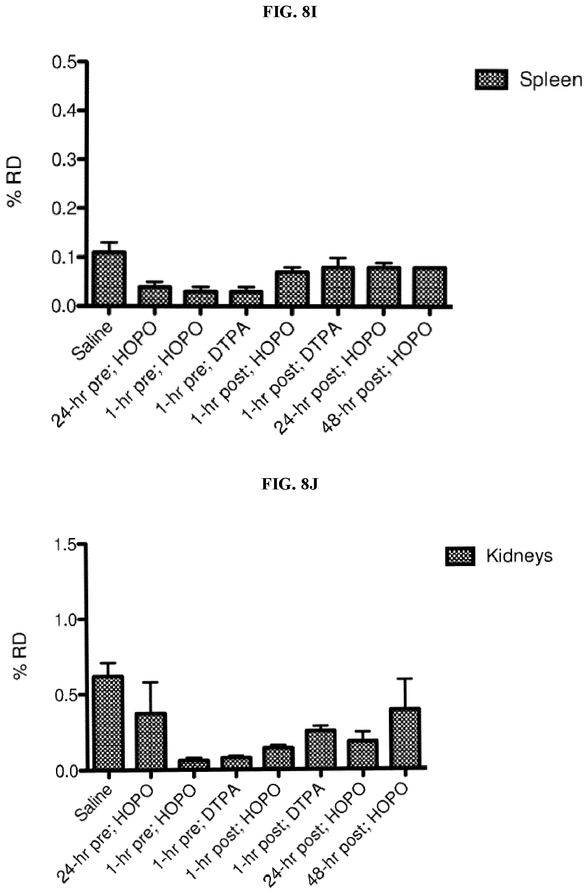

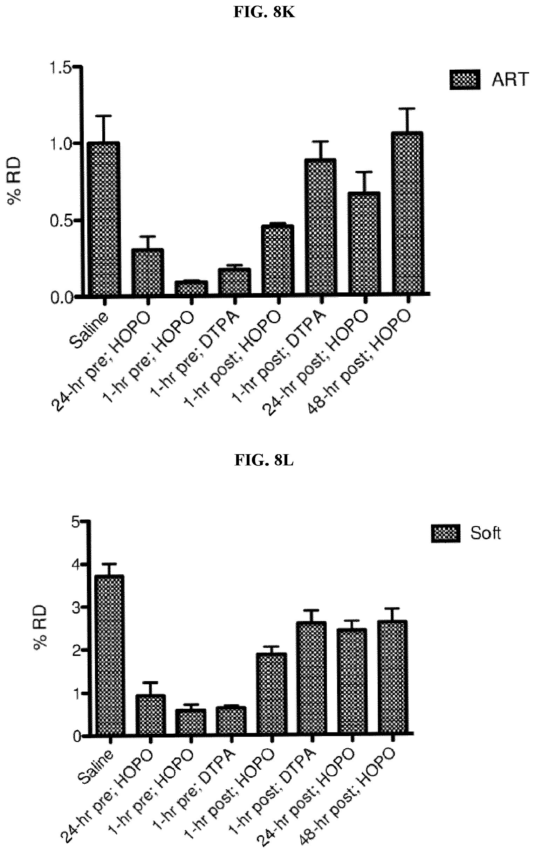

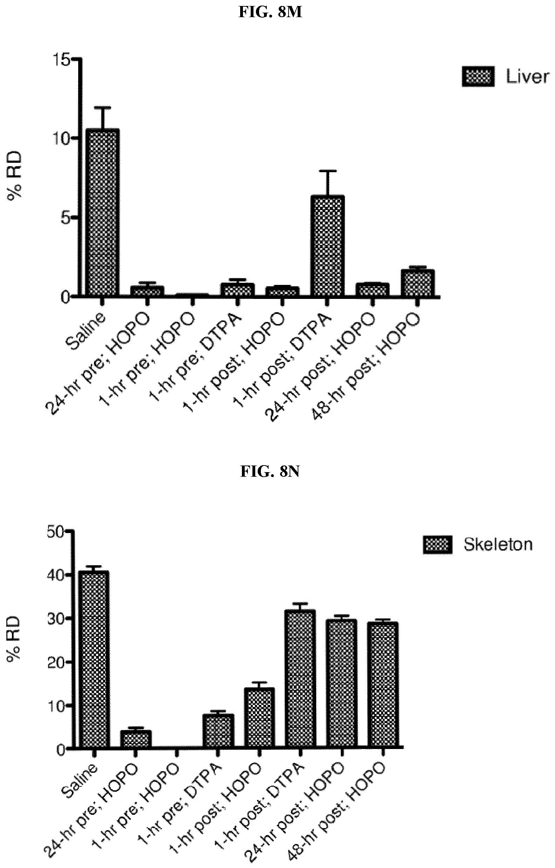

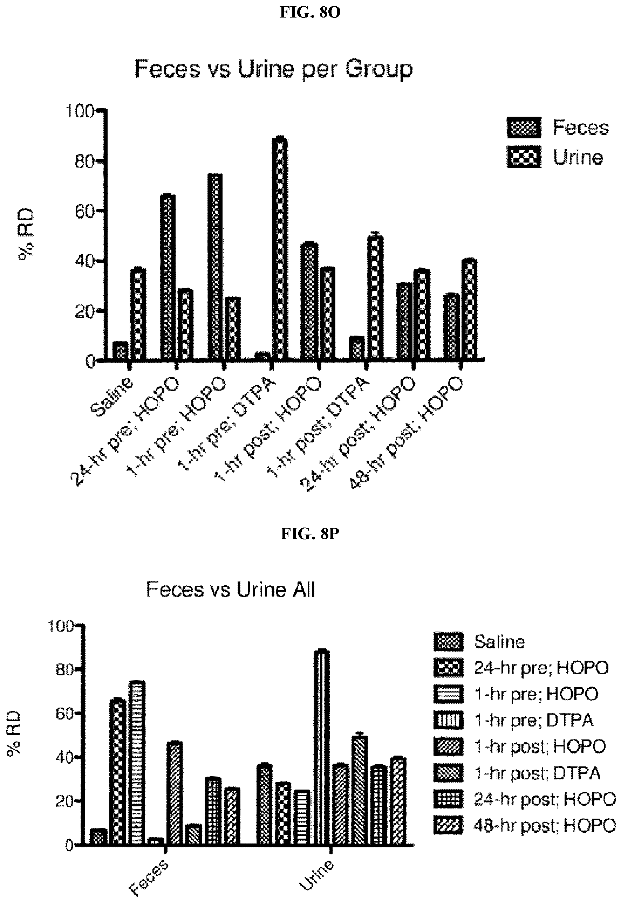

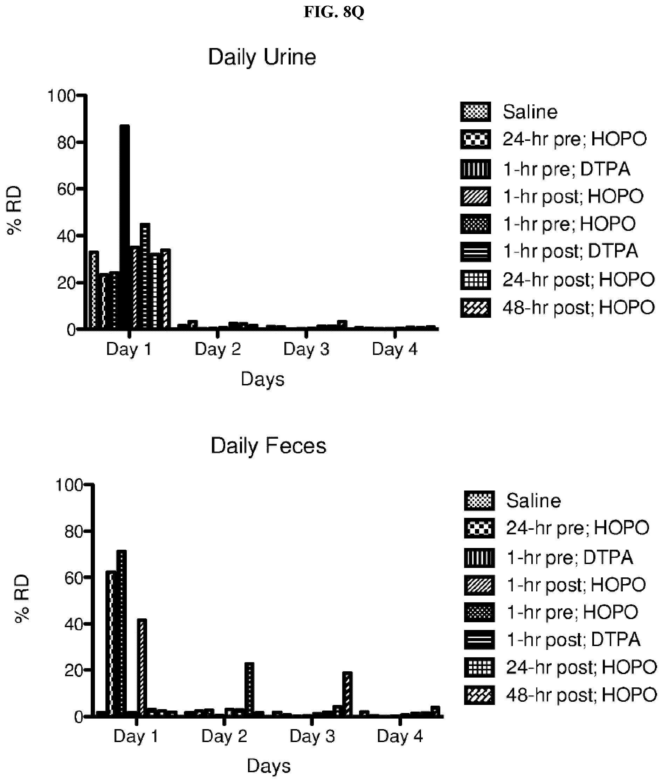

he results of the various test arrangements in Table 9.1 are detailed in Figures. 8A-8Q. The data is plotted as a percent of recovered dose (%RD), with whole animal and specific tissue isotope content determine at a unique time point (4 days post metal challenge) while excreta are determined daily until necropsy. The results demonstrated the efficacy of 3,4,3-LI(1,2-HOPO) at removing Gd-153 from mice when injected both prophylactically and post-exposure. Indeed, even a dose administered up to 24 hours prophylactically resulted in a high and almost quantitative level of decorporation. When administered as late as 48 hours post-exposure, 3,4,3-LI(1,2-HOPO) was extremely efficient at removing Gd-153, with similar amounts of removed Gd-153 as when treatment is performed only 1 hour post-exposure with DTPA. 3,4,3-LI(1,2-HOPO) is more efficient than DTPA at removing Gd-153 from kidneys, an important feature for patients with preconditions such as impaired kidney function. The almost-exclusively-fecal excretion pathway of 3,4,3-LI(1,2-HOPO) is also remarkably different from that of DTPA (urinary). One additional significant advantage of 3,4,3-LI(1,2-HOPO) is its ability at removing Gd-153 from the skeleton. FIGs. 8A-8C depict a general overview of the results. FIG. 8D depicts a comparative analysis of the recovered dose in the body vs excreta. FIGs. 8E-8P depict a break down by tissue type, including brain (FIG. 8E), Thymus (FIG. 8F), heart (FIG. 8G), lungs (FIG. 8H), spleen (FIG. 8I), kidneys (FIG. 8J), abdominal remaining tissue (ART, FIG. 8K), all other soft tissues (SOFT, FIG. 8L), liver (FIG. 8M), and skeleton (SKEL, FIG. 8N). FIGs. 8O and 8P show the total urinary and fecal excretion at the time of necropsy. FIG. 8Q depicts the daily excreta. As can be seen throughout the various figures, Gd decorporation can be achieved both pre and post exposure to Gd.

It is to be understood that, while the invention has been described in conjunction with the preferred specific embodiments thereof, the foregoing description is intended to illustrate and not limit the scope of the invention. Other aspects, advantages, and modifications within the scope of the invention will be apparent to those skilled in the art to which the invention pertains.

All patents, patent applications, and publications mentioned herein are hereby incorporated by reference in their entireties.

### Exemplary Embodiments.

The following example embodiments identify some possible permutations of combinations of features disclosed herein.
1 A method for treating a subject for a heavy metal exposure, the method comprising administering a therapeutically effective amount of a pharmaceutical composition comprising a 1,2-HOPO chelating agent to a subject that has an excess amount of one or more of gadolinium, lead, tin, yttrium, scandium, or cadmium, wherein administering results in decorporating, clearing or reducing the amount of gadolinium, lead, tin, or cadmium from the subject.
2. The method of item 1, wherein the subject has been exposed to, have been in contact with, or contaminated by one or more actinides and/or lanthanides, or a mixture thereof.
3. The method of item 1, wherein the administering step results in decorporating, clearing or reducing the amount of actinide and/or lanthanide, or both from one or more systems or organs of the subject.
4. The method of item 1, wherein the 1,2-HOPO chelating agent is defined by the structure: 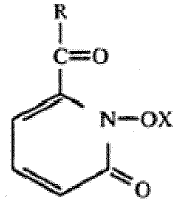 wherein R is a hydroxy group or 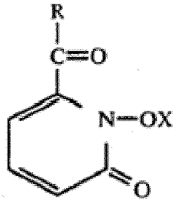 where R₁ and R₂ are selected from the group consisting of H, --CH₃,-CH₂CH₃ and --CH₂--ϕ, and X is either hydrogen, an alkali metal ion, or a quaternary ammonium ion.
5. The method of item 1, wherein the 1,2-HOPO chelating agent is defined by one molecule selected from the group consisting of: 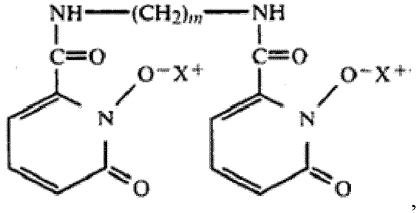 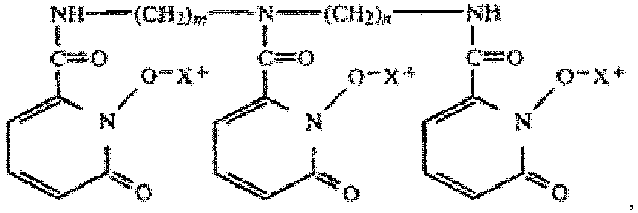 and 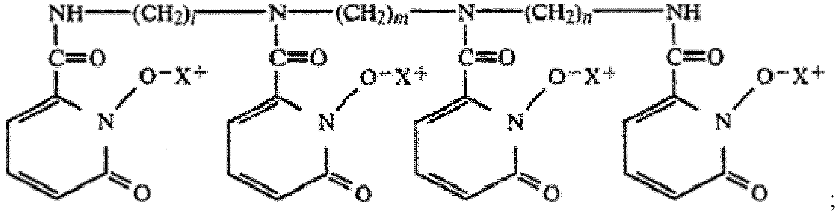 wherein l, m and n are integers between one and twenty.
6. The method of item 5, wherein m is three.
7. The method of item 5, wherein n is four.
8. The method of item 5, wherein l and n are three, and m is four.
9. The method of item 1, wherein the 1,2-HOPO chelating agent is 3,4,3-LI-1,2-HOPO.
10. The method of item 1, wherein the subject has an excess amount of one or more of gadolinium, lead, yttrium, scandium, cadmium, or tin.
11. A method for the prophylactic treatment of a subject for metal exposure, the method comprising administering a therapeutically effective amount of a pharmaceutical composition comprising a 1,2-HOPO chelating agent to a subject.
12. The method of item 11, wherein the 1,2-HOPO chelating agent is 3,4,3-LI-1,2-HOPO.
13. The method of item 11, wherein the metal is a heavy metal.
14. The method of item 13, wherein the heavy metal is selected from the group consisting of gadolinium, lead, tin, cadmium, yttrium, scandium, and plutonium.
15. The method of item 14, wherein the metal is an actinide a lanthanide, or a mixture thereof.
16. A method comprising:
   administering a 1,2-HOPO chelating agent to a subject prior to or after administering a MRI contrast agent to the subject.
17. The method of item 16, wherein the subject is identified as one that is to receive the MRI contrast agent.
18. The method of item 16, wherein the contrast agent comprises Gd.
19. The method of item 18, wherein an amount of the contrast agent is from 100-600 µmol/kg.
20. The method of item 16, wherein the 1,2-HOPO chelating agent is administered prior to the subject receiving the MRI contrast agent.
21. The method of item 16, wherein the 1,2-HOPO chelating agent is administered after the subject received the MRI contrast agent.
22. The method of item 16, wherein the 1,2-HOPO chelating agent is 3,4,3-LI-1,2-HOPO.
23. The method of item 16, wherein the subject has severely impaired kidney function.

## Claims

1. A pharmaceutical composition comprising a 1,2-hydroxypyridonate (HOPO) chelating agent for use in a medicament for treating of a heavy metal exposure by administering a therapeutically effective amount of the pharmaceutical composition to the subject that has an excess amount of the heavy metal, wherein administering results in decorporating, clearing, or reducing the amount the heavy metal.

2. The pharmaceutical composition for use in a medicament for treating of a heavy metal exposure according to claim 1, wherein the heavy metal is one or more of plutonium, americium, lead, cadmium, tin, an actinide, a lanthanide, a transition metal, a metalloid, a metallic element within Groups 13, 14, and 15 of the Periodic Table, or a mixture thereof.

3. The pharmaceutical composition for use in a medicament for treating of a heavy metal exposure according to any one of claims 1-2, wherein the subject has been exposed to, have been in contact with, or contaminated by heavy metal.

4. The pharmaceutical composition for use in a medicament for treating of a heavy metal exposure according to any one of claims 1-3, wherein the administering step results in decorporating, clearing, or reducing the amount the heavy metal.

5. The pharmaceutical composition for use in a medicament for treating of a heavy metal exposure according to any one of claims 1-4, wherein the 1,2-hydroxypyridonate (HOPO) chelating agent is defined by the structure: 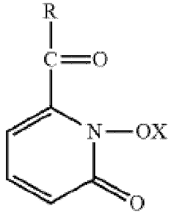 wherein R is a hydroxy group or 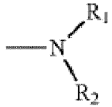 where R₁ and R₂ are selected from the group consisting of H, --CH₃,-CH₂CH₃ and --CH₂--ϕ, and X is either hydrogen, an alkali metal ion, or a quaternary ammonium ion.

6. The pharmaceutical composition for use in a medicament for treating of a heavy metal exposure according to any one of claims 1-5, wherein the 1,2-hydroxypyridonate (HOPO) chelating agent is defined by one molecule selected from the group consisting of: 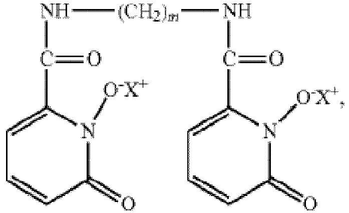 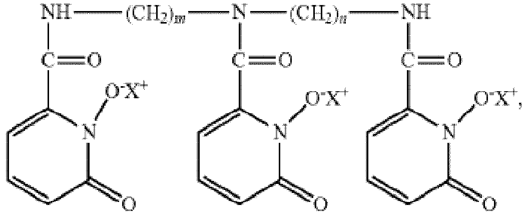 and 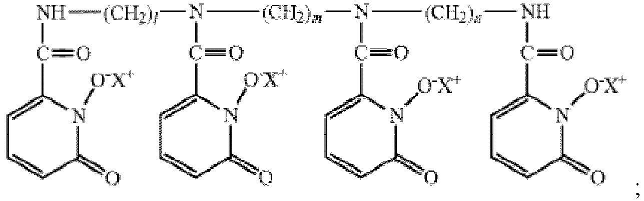 wherein l, m and n are integers between one and twenty, and wherein X is either hydrogen, an alkali metal ion, or a quaternary ammonium ion.

7. The pharmaceutical composition for use in a medicament for treating of a heavy metal exposure according to any one of claims 1-6, wherein m is three.

8. The pharmaceutical composition for use in a medicament for treating of a heavy metal exposure according to any one of claims 1-6, wherein at least one of:
(a) n is four; or
(b) l and n are three, and m is four.

9. The pharmaceutical composition for use in a medicament for treating of a heavy metal exposure according to any one of claims 1-8, wherein the 1,2-hydroxypyridonate (HOPO) chelating agent is 3,4,3-LI-1,2-HOPO.

10. A pharmaceutical composition comprising a 1,2-hydroxypyridonate (HOPO) chelating agent for use in a prophylactic treatment of a subject for a heavy metal exposure by administering a therapeutically effective amount of the pharmaceutical composition to the subject.

11. The pharmaceutical composition for use in a prophylactic treatment according to claim 10, wherein 1,2-hydroxypyridonate (HOPO) chelating agent is 3,4,3-LI-1,2-HOPO.

12. The pharmaceutical composition for use in a prophylactic treatment according to any one of claims 10-11, wherein the heavy metal is one or more of plutonium, americium, lead, cadmium, tin, an actinide, a lanthanide, a transition metal, a metalloid, a metallic element within Groups 13, 14, and 15 of the Periodic Table, or a mixture thereof.

13. A 1,2-hydroxypyridonate (HOPO) chelating agent for use in reducing a risk associated with MRI contrast agents by administering the chelating agent prior to or after administering an MRI contrast agent comprising gadolinium to a subject.

14. The chelating agent for use in reducing a risk according to claim 13, wherein at least one of:
(a) the subject is identified as one that is to receive the MRI contrast agent;
(b) the 1,2-HOPO chelating agent is administered prior to the subject receiving the MRI contrast agent;
(c) the 1,2-HOPO chelating agent is administered after the subject received the MRI contrast agent;
(d) the 1,2-HOPO chelating agent is 3,4,3-LI-1,2-HOPO; or
(e) the subject has severely impaired kidney function.

15. The chelating agent for use in reducing a risk according to claim 14, wherein an amount of the contrast agent is from 100-600 µmol/kg.
